(19) Europäisches Patentamt / European Patent Office / Office européen des brevets

(11) **EP 4 394 781 A1**

(12) # EUROPEAN PATENT APPLICATION
published in accordance with Art. 153(4) EPC

(43) Date of publication:
**03.07.2024 Bulletin 2024/27**

(21) Application number: **23851324.6**

(22) Date of filing: **26.05.2023**

(51) International Patent Classification (IPC):
**G16C 20/30** (2019.01)

(86) International application number:
**PCT/CN2023/096605**

(87) International publication number:
**WO 2024/032096 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.08.2022 CN 202210952642**

(71) Applicant: **Tencent Technology (Shenzhen) Company Limited**
**Shenzhen, Guangdong, 518057 (CN)**

(72) Inventors:
• **ZHAO, Peilin**
  **Shenzhen, Guangdong 518057 (CN)**
• **YU, Yang**
  **Shenzhen, Guangdong 518057 (CN)**
• **LU, Chan**
  **Shenzhen, Guangdong 518057 (CN)**

(74) Representative: **Gevers Patents**
**De Kleetlaan 7A**
**1831 Diegem (BE)**

(54) **REACTANT MOLECULE PREDICTION METHOD AND APPARATUS, TRAINING METHOD AND APPARATUS, AND ELECTRONIC DEVICE**

(57) The present application relates to the technical field of backward reactions in the field of chemistry, and provides a reactant molecule prediction method and apparatus, a training method and apparatus, and an electronic device. The method comprises: extracting features of a product molecule to obtain the features of the product molecule; on the basis of the features of the product molecule, predicting a conversion path from the product molecule to a plurality of reactant molecules by using a backward reaction prediction model; and predicting synthons and completed synthons according to the conversion path to obtain the plurality of reactant molecules corresponding to the product molecule. The method provided by the present application can reduce the prediction complexity of reactant molecules, improve the generalization performance of reactant molecule prediction, and also can improve the prediction performance of the reactant molecules.

FIG. 2

**Description**

This application claims priority to Chinese Patent Application No. 202210952642.6, entitled "METHOD FOR PREDICTING REACTANT MOLECULE, TRAINING METHOD, APPARATUS, AND ELECTRONIC DEVICE" filed on August 09, 2022.

FIELD OF THE TECHNOLOGY

Embodiments of this application relate to the field of chemical reverse reactions, and more specifically, to a method for predicting a reactant molecule, a training method, an apparatus, and an electronic device.

BACKGROUND OF THE DISCLOSURE

Retrosynthetic reactant prediction in organic chemistry is a key step in development of new drugs and manufacturing of new materials, to find a group of commercially available reactant molecules for synthesis of a product molecule. In a conventional method, reactant molecules are obtained by matching reaction templates and reactant molecules. However, the reaction templates need to be manually extracted by professional researchers, a process thereof is very time-consuming, and the reflection templates cannot cover all reaction types. In recent years, development of a deep learning technology makes it possible to learn potential reaction types from large databases of organic chemical reactions. Therefore, constructing a powerful reverse reaction prediction model by using the deep learning technology is particularly important.

Up to now, there are generally two types of model structures commonly used. One is a sequence translation model indicated based on a sequence of a simplified molecular input line entry system (SMILES), where the SMILES is a specification configured for clearly describing molecular structures. The other one is a graph generation model indicated based on graphs.

The graph generation model generally divides an organic chemical reverse reaction prediction task into two sub-tasks, namely, synthon recognition and synthon completion. Generally, a graph neural network may be constructed to recognize a synthon to obtain a synthon of a product molecule, and a graph variational autoencoder may be constructed to complete the synthon atom by atom. However, by constructing two independent networks respectively for synthon recognition and synthon completion, prediction complexity is increased, and good generalization performance cannot be achieved because the two sub-tasks have different optimization targets. In addition, complexity of completing the synthon atom by atom is high, limiting prediction performance.

SUMMARY

Embodiments of this application provide a method for predicting a reactant molecule, a training method, an apparatus, and an electronic device, which can reduce reactant molecule prediction complexity and improve generalization performance of reactant molecule prediction, and can also improve reactant molecule prediction performance.

According to a first aspect, this application provides a method for predicting a reactant molecule, including:

performing feature extraction on a product molecule, to obtain a feature of the product molecule;

predicting a conversion path between the product molecule and a set of to-be-predicted reactant molecules by inputting the feature of the product molecule into a reverse reaction prediction model, the conversion path including an editing sequence and a synthon completion sequence, the editing sequence comprising a plurality of editing actions each indicating both a constituent of the product molecule and a target state of the constituent, the constituent being an atom or a chemical bond of the product molecule, the synthon completion sequence comprising a plurality of synthon complete actions each indicating both an interface atom and a corresponding motif, the motif comprising a plurality of atoms or atomic edges for connecting the plurality of atoms;

performing the plurality of editing actions to edit the respective constituent into the corresponding target state indicated by the respective editing action, in order to obtain a plurality of synthons corresponding to the product molecule; and

performing, for each of the plurality of synthons, at least one synthon completion action corresponding to the respective synthon, to add the motif indicated by the at least one corresponding synthon completion action according to the corresponding interface atom, to obtain a plurality of predicted reactant molecules each corresponding to a respective one of the plurality of synthons.

According to a second aspect, this application provides a method for training a reverse reaction prediction model, including:

performing feature extraction on a product molecule, to obtain a feature of the product molecule;

predicting a conversion path between the product molecule and a plurality of reactant molecules by inputting the feature of the product molecule into the reverse reaction prediction model,

the conversion path including an editing sequence and a synthon completion sequence; each editing action in the editing sequence being configured for indicating an constituent and a target state, and the constituent being an atom or a chemical bond in the product molecule; and for a plurality of synthons of the product molecule obtained by using the editing sequence, the synthon completion sequence including at least one synthon completion action corresponding to each synthon in the plurality of synthons, each synthon completion action in the at least one synthon completion action being configured for indicating a motif and an interface atom, and the motif including a plurality of atoms or atomic edges for connecting the plurality of atoms; and

training the reverse reaction prediction model based on a loss between the conversion path and a training path.

According to a third aspect, this application provides an apparatus for predicting a reactant molecule, including:

an extraction unit, configured to perform feature extraction on a product molecule, to obtain a feature of the product molecule;

a prediction unit, configured to predict a conversion path between the product molecule and a set of to-be-predicted reactant molecules by inputting the feature of the product molecule into a reverse reaction prediction model, the conversion path including an editing sequence and a synthon completion sequence, the editing sequence comprising a plurality of editing actions each indicating both a constituent of the product molecule and a target state of the constituent, the constituent being an atom or a chemical bond of the product molecule, the synthon completion sequence comprising a plurality of synthon complete actions each indicating both an interface atom and a corresponding motif, the motif comprising a plurality of atoms or atomic edges for connecting the plurality of atoms;

an editing unit, configured to perform the plurality of editing actions to edit the respective constituent into the corresponding target state indicated by the respective editing action, in order to obtain a plurality of synthons corresponding to the product molecule; and

an addition unit, configured to perform, for each of the plurality of synthons, at least one synthon completion action corresponding to the respective synthon, to add the motif indicated by the at least one corresponding synthon completion action according to the corresponding interface atom, to obtain a plurality of predicted reactant molecules each corresponding to a respective one of the plurality of synthons.

According to a fourth aspect, this application provides an apparatus for training a reverse reaction prediction model, including:

an extraction unit, configured to perform feature extraction on a product molecule, to obtain a feature of the product molecule;

a prediction unit, configured to predict a conversion path between the product molecule and a plurality of reactant molecules by inputting the feature of the product molecule into the reverse reaction prediction model,

the conversion path including an editing sequence and a synthon completion sequence; each editing action in the editing sequence being configured for indicating a constituent and a target state, and the constituent being an atom or a chemical bond in the product molecule; and for a plurality of synthons of the product molecule obtained by using the editing sequence, the synthon completion sequence including at least one synthon completion action corresponding to each synthon in the plurality of synthons, each synthon completion action in the at least one synthon completion action being configured for indicating a motif and an interface atom, and the motif including a plurality of atoms or atomic edges for connecting the plurality of atoms; and

a training unit, configured to train the reverse reaction prediction model based on a loss between the conversion path and a training path.

According to a fifth aspect, this application provides an electronic device, including:

a processor, suitable for implementing computer instructions; and

a computer-readable storage medium, storing computer instructions, the computer instructions being suitable for being loaded by the processor to perform the method for predicting a reactant molecule according to the first aspect or the method for training a reverse reaction prediction model according to the second aspect.

According to a sixth aspect, an embodiment of this application provides a computer-readable storage medium, storing computer instructions, the computer instructions, when read and executed by a processor of a computer device, enabling the computer device to perform the method for predicting a reactant molecule according to the first aspect or the method for training a reverse reaction prediction model according to the second aspect.

According to a seventh aspect, an embodiment of this application provides a computer program product or a computer program, the computer program product or the computer program including computer instructions, the computer instructions being stored in a computer-readable storage medium. A processor of a computer device reads the computer instructions from the computer-readable storage medium, and the processor executes the computer instructions, to enable the computer device to perform the method for predicting a reactant molecule according to the first aspect or the method for training a reverse reaction prediction model according to the second aspect.

Based on the foregoing technical solutions, by introducing a reverse reaction prediction model configured to predict a conversion path between a product molecule and a plurality of reactant molecules, a synthon prediction task and a synthon completion prediction task can be merged. In other words, the reverse reaction prediction model introduced in the embodiments of this application can learn a potential relationship between two sub-tasks: synthon prediction and synthon completion. In this way, generalization performance of the model is greatly improved, reactant molecule prediction complexity can be reduced, and generalization performance of reactant molecule prediction can be improved. In addition, by introducing a motif and designing the motif as a structure including a plurality of atoms or atomic edges for connecting the plurality of atoms, a short and accurate conversion path can be reasonably constructed. In this way, an excessively long length of a synthon completion sequence is avoided, reactant molecule prediction difficulty is reduced, and reactant molecule prediction accuracy is improved, so that reactant molecule prediction performance can be improved.

In addition, by improving the reactant molecule prediction performance, the following technical effects can also be obtained:

1. Synthetic paths may be planned for designed drugs or new material molecules, thereby improving research efficiency of the drugs or the new material molecules.

2. Some potential scientific laws may be revealed, thereby providing new scientific knowledge.

3. More accurate synthesis route plans than those of professional researchers may be provided. In this way, reliable reactant molecules can be predicted in the absence of reaction templates, and reaction types that have not been clearly defined by the professional researchers can also be predicted, thereby greatly improving research and development efficiency of new drugs and new materials.

BRIEF DESCRIPTION OF THE DRAWINGS

FIG. 1 shows an example of a system architecture according to an embodiment of this application.

FIG. 2 is a schematic flowchart of a method for predicting a reactant molecule according to an embodiment of this application.

FIG. 3 shows an example of a conversion path according to an embodiment of this application.

FIG. 4 is another schematic flowchart of a method for predicting a reactant molecule according to an embodiment of this application.

FIG. 5 is a schematic flowchart of a method for training a reverse reaction prediction model according to an embodiment of this application.

FIG. 6 is a schematic block diagram of an apparatus for predicting a reactant molecule according to an embodiment of this application.

FIG. 7 is a schematic block diagram of an apparatus for training a reverse reaction prediction model according to an embodiment of this application.

FIG. 8 is a schematic block diagram of an electronic device according to an embodiment of this application.

DESCRIPTION OF EMBODIMENTS

The following clearly and completely describes technical solutions in the embodiments of this application with reference to the accompanying drawings in the embodiments of this application.

The solutions provided in this application may relate to the technical field of artificial intelligence (AI).

Specifically, the solutions provided in this application may relate to the technical field of performing reverse reaction prediction based on AI in the field of chemistry.

AI is a theory, method, technology and application system that uses digital computers or machines controlled by digital computers to simulate, extend and expand human intelligence, perceive the environment, acquire knowledge, and use knowledge to obtain the best results. In other words, AI is a comprehensive technology in computer science, and attempts to understand the essence of intelligence and produce a new intelligent machine that can react in a manner similar to human intelligence. AI is to study design principles and implementation methods of various intelligent machines, so that the machines can perceive, infer, and make decisions.

It is to be understood that, an AI technology is a comprehensive discipline, covering a wide range of fields including both a hardware-level technology and a software-level technology. Basic AI technologies generally include technologies such as a sensor, a dedicated AI chip, cloud computing, distributed storage, a big data processing technology, an operating/interaction system, and electromechanical integration. AI software technologies mainly include a computer vision technology, a speech processing technology, a natural language processing technology, machine learning/deep learning, and the like.

With the research and progress of the AI technology, the AI technology is studied and applied in a plurality of fields such as a common smart home, a smart wearable device, a virtual assistant, a smart speaker, smart marketing, unmanned driving, automatic driving, an unmanned aerial vehicle, a robot, smart medical care, and smart customer service. It is believed that with the development of technologies, the AI technology will be applied to more fields, and play an increasingly important role.

The embodiments of this application may relate to computer vision (CV) technologies in the AI technology.

Specifically, the embodiments of this application relate to the technical field of performing reverse reaction prediction based on CV-recognized result in the field of chemistry.

CV is a science field that studies how to use a machine to "see", and furthermore, that uses a camera and a computer to replace human eyes to perform machine vision such as recognition, prediction, and measurement on an object, and further perform graphic processing, so that the computer processes the object into an image more suitable for human eyes to observe, or an image transmitted to an instrument for detection. As a scientific discipline, the CV studies related theories and technologies and attempts to establish an AI system that can obtain information from images or multidimensional data. The CV technologies generally include technologies such as image processing, image recognition, image semantic understanding, image retrieval, OCR, video processing, video semantic understanding, video content/behavior recognition, 3D object reconstruction, a 3D technology, virtual reality, augmented reality, synchronous positioning, and map construction, and further include biometric feature recognition technologies such as common face recognition and fingerprint recognition.

The embodiments of this application may also relate to machine learning (ML) in the AI.

Specifically, the embodiments of this application may relate to the technical field of performing reverse reaction prediction by using an ML prediction model.

ML is a multi-field interdiscipline, and relates to a plurality of disciplines such as the probability theory, statistics, the approximation theory, convex analysis, and the algorithm complexity theory. The ML specializes in studying how a computer simulates or implements a human learning behavior to obtain new knowledge or skills, and reorganize an existing knowledge structure to keep improving its performance. The ML is a core of the AI, is a basic way to make the computer intelligent, and is applied to various fields of the AI. The ML and deep learning generally include technologies such as an artificial neural network, a belief network, reinforcement learning, transfer learning, inductive learning, and learning from demonstrations.

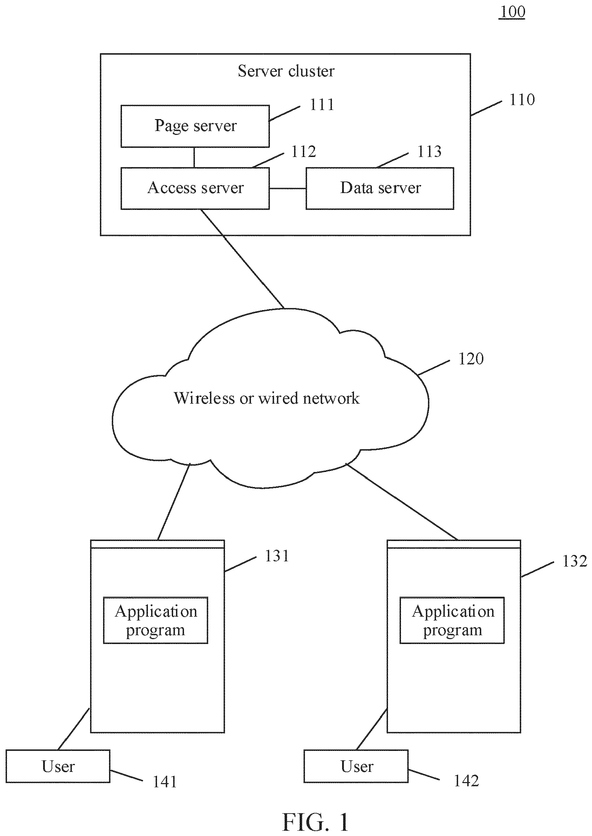

FIG. 1 shows an example of a system architecture 100 according to an embodiment of this application.

The system architecture 100 may be an application program system, and a specific type of an application program is not limited in embodiments of this application. |The system architecture 100 includes: a terminal 131, a terminal 132, and a server cluster 110. The terminal 131 and the terminal 132 each are connected to the server cluster 110 through a wireless or wired network 120.

The terminal 131 and the terminal 132 may be at least one of a smartphone, a game console, a desktop computer, a tablet computer, an ebook reader, an MP3 player, an MP4 player, and a portable laptop computer. An application program is installed and run on the terminal 131 and the terminal 132. The application program may be any one of an online video program, a short video program, a picture sharing program, a sound social program, an animation program, a wallpaper program, a news push program, a supply and demand information push program, an academic exchange program, a technical exchange program, a policy exchange program, a program including a comment mechanism, a program including an opinion publishing mechanism, and a knowledge sharing program. The terminal 131 and the terminal 132 may be terminals respectively used by a user 141 and a user 142, and the application program run on the terminal 131 and the terminal 132 logs in to a user account.

The server cluster 110 includes at least one of one server, a plurality of servers, a cloud computing platform, and a virtualization center. The server cluster 110 is configured to provide a backend service for an application program (for example, the application program run on the first terminal 131 and the second terminal 132). In one embodiment, the server cluster 110 is responsible for primary computing work, and the terminal 131 and the terminal 132 are responsible for secondary computing work; or the server cluster 110 is responsible for secondary computing work, and the terminal 131 and the terminal 132 are responsible for primary computing work; or the terminal 131, the terminal 132, and the server cluster 110 perform collaborative computing by using a distributed computing architecture between each other. For example, computing work related to this application may be computing work or auxiliary work related to retrosynthetic reactant prediction in organic chemistry.

In one embodiment, by using an example in which the system architecture 100 is a web browsing system, the server cluster 110 includes: an access server 112, a web page server 111, and a data server 113. There may be one or more access servers 112. The access servers 112 may be deployed in different cities nearby. The access servers 112 are configured to receive service requests from the terminal 131 and the terminal 132, and forward the service requests to corresponding servers to deal with. The web page server 111 is a server configured to provide a web page to the terminal 131 and the terminal 132. Tracking code is integrated into the web page. The data server 113 is configured to receive data (for example, service data) reported by the terminal 131 and the terminal 132.

Retrosynthetic reactant prediction in organic chemistry is a key step in development of new drugs and manufacturing of new materials, to find a group of commercially available reactant molecules for synthesis of a product molecule. In a conventional method, reactant molecules are obtained by matching reaction templates and reactant molecules. However, the reaction templates need to be manually extracted by professional researchers, a process thereof is very time-consuming, and the reflection templates cannot cover all reaction types. In recent years, development of a deep learning technology makes it possible to learn potential reaction types from large databases of organic chemical reactions. Therefore, constructing a powerful reverse reaction prediction model by using the deep learning technology is particularly important.

Up to now, there are generally two types of model structures commonly used. One is a sequence translation model indicated based on a sequence of a simplified molecular input line entry system (SMILES), where the SMILES is a specification configured for clearly describing molecular structures. The other one is a graph generation model indicated based on graphs.

The graph generation model generally divides an organic chemical reverse reaction prediction task into two sub-tasks, namely, synthon recognition and synthon completion. Generally, a graph neural network may be constructed to recognize a synthon to obtain a synthon of a product molecule, and a graph variational autoencoder may be constructed to complete the synthon atom by atom. However, by constructing two independent networks respectively for synthon recognition and synthon completion, prediction complexity is increased, and good generalization performance cannot be achieved because the two sub-tasks have different optimization targets. In addition, complexity of completing the synthon atom by atom is high, limiting prediction performance.

In view of the foregoing problems, it may be considered that a pre-extracted leaving group is used to complete the synthon, to reduce synthon completion difficulty, thereby improving reverse reaction prediction performance. The leaving group is an atom or a functional group that is separated from a large molecule during a chemical reaction. The functional group is an atom or an atomic group that determines a chemical property of an organic compound. Common functional groups include a carbon-carbon double bond, a carbon-carbon triple bond, a hydroxyl group, a carboxyl group, an ether bond, an aldehyde group, and the like.

However, the pre-extracted leaving group is used to complete the synthon, which does not improve generalization performance of the model. In addition, limited by a characteristic of the leaving group, a distribution of samples is extremely unbalanced, limiting a prediction effect of the reactant molecules.

In addition, the two sub-tasks may be jointly learned through an end-to-end model, in other words, the two sub-tasks, namely, synthon recognition and synthon completion, are integrated into the end-to-end model, and the synthon is completed atom by atom or benzene ring by benzene ring. The end-to-end model is a model that may perform training optimization through a single optimization target.

However, although an architecture of the end-to-end model enables the model to achieve better generalization performance, using small units such as a single atom and a benzene ring to complete the synthon needs to predict a longer graph editing sequence, which increases prediction difficulty and then makes accuracy of a top-1 category accuracy unable to reach a latest state of the art (sota). The accuracy of the top-1 category accuracy is accuracy that the top-1 category matches an actual result.

In view of this, the embodiments of this application provide a method for predicting a reactant molecule, a training method, an apparatus, and an electronic device, which can reduce reactant molecule prediction complexity and improve generalization performance of reactant molecule prediction, and can also improve reactant molecule prediction performance. Specifically, in the embodiments of this application, an end-to-end reverse reaction prediction model is designed, to jointly optimize two sub-tasks, namely, synthon prediction and synthon completion. The synthon prediction task may be constructed as predicting an editing sequence that can represent a conversion process from a product molecule to synthons. The synthon completion prediction task may be constructed as predicting a synthon completion sequence that can represent a conversion process from the synthons to reactant molecules, and constructing, through the editing sequence and the synthon completion sequence, a conversion path that can represent a conversion process between from the product molecule and the reactant molecules.

As a data amount increases, the solutions provided in the embodiments of this application can be easily extended to more complex and diverse chemical reaction models. For example, the reverse reaction prediction model provided in the embodiments of this application may be extended to a multi-step reverse reaction prediction task. For example, a Monte Carlo tree search algorithm or the like may be used to extend the reverse reaction prediction model provided in the embodiments of this application to the multi-step reverse reaction prediction task.

In addition, motifs are also introduced in the embodiments of this application, which can greatly avoid a limited prediction effect of the reactant molecules caused by extremely unbalanced samples in a leaving group, and resolve a problem of excessively high prediction complexity when using atoms to complete the synthons, thereby improving prediction accuracy of the reverse reaction prediction model.

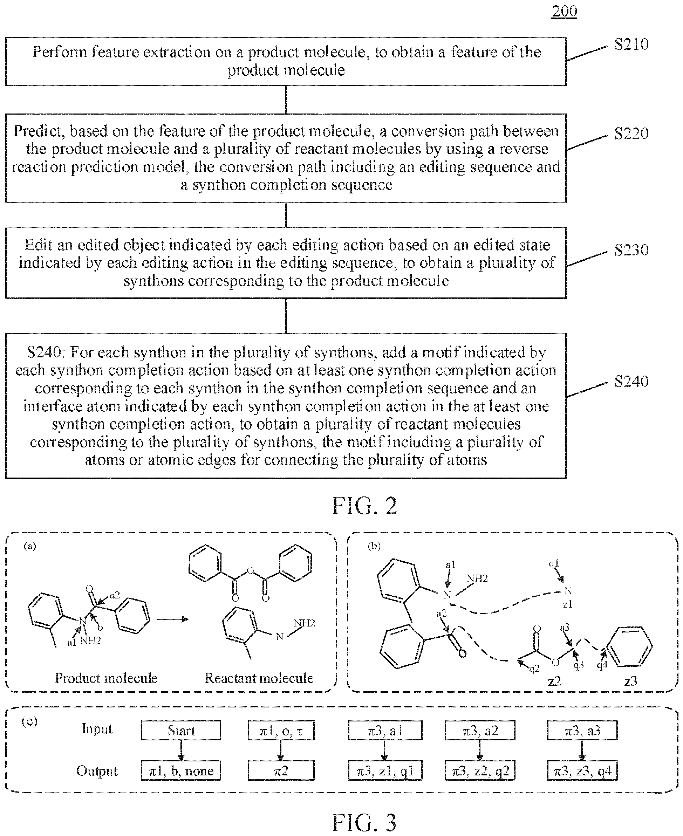

FIG. 2 is a schematic flowchart of a method 200 for predicting a reactant molecule according to an embodiment of this application. The prediction method 200 may be performed by any electronic device having a data processing capability. For example, the electronic device may be implemented as a server. The server may be an independent physical server, or may be a server cluster including a plurality of physical servers or a distributed system, or may be a cloud server providing basic cloud computing services, such as a cloud service, a cloud database, cloud computing, a cloud function, cloud storage, a network service, cloud communication, a middleware service, a domain name service, a security service, big data, and an artificial intelligence platform. The server may be connected directly or indirectly through a wired or wireless communication method, which is not limited in this application. For ease of description, the prediction method provided in this application is described below by using an apparatus for predicting a reactant molecule as an example.

As shown in FIG. 2, the prediction method 200 includes the following steps.

S210: Perform feature extraction on a product molecule, to obtain a feature of the product molecule.

For example, feature extraction is performed on the product molecule by using a simplified molecular input line entry system (SMILES) or a graph neural network (GNN), to obtain the feature of the product molecule. Certainly, feature extraction may alternatively be performed on the product molecule by using another model having a type function or a framework. This is not specifically limited in embodiments of this application.

For example, the feature of the product molecule may be determined based on a feature of each atom in the product molecule. For example, a global attention pooling function may be used to calculate features of all atoms to obtain the feature of the product molecule.

For example, an original feature of each atom and an original feature of a chemical bond between each atom and a neighbor node of each atom may be encoded, to obtain the feature of each atom. For example, a MPNN may be used to encode the original feature of each atom and the original feature of the chemical bond between each atom and the neighbor node of each atom, to obtain the feature of each atom. The original feature of each atom is configured for representing at least one of the following information: an atom type (such as C, N, O, or S), a degree of a chemical bond, chirality, a quantity of hydrogen atoms, and the like. The original feature of the chemical bond is configured for representing at least one of the following information: a chemical bond type (such as a single bond, a double bond, a triple bond, or an aromatic bond), a configuration, aromaticity, and the like.

For example, the feature of the product molecule may further include a feature configured for indicating chemical bond types in the product molecule. For example, the chemical bond types in the product molecule include, but not limited to: a single bond, a double bond, a triple bond, and no chemical bond.

For example, the feature of the product molecule may be embodied in a form of a vector or a matrix. Certainly, the feature of the product molecule may alternatively be embodied in a form of an array or information in another format. This is not specifically limited in this application.

The feature of the product molecule is illustratively described below by using a feature vector as an example.

For example, a molecule including $n$ atoms and $m$ chemical bonds may be indicated as a graph structure $G = (V, E)$, where $V$ is a set of atoms with a size of $n$, and E is a set of chemical bonds with a size of $m$. Each atom $v \in V$ has a feature vector $x_v$ indicating information such as an atom type (such as C, N, O, or S), a degree of a chemical bond, chirality, and a quantity of hydrogen atoms. Similarly, each chemical bond $e \in E$ has a feature vector $x_{v,u}$ including information such as a chemical bond type (such as a single bond, a double bond, a triple bond, or an aromatic bond), a configuration, and aromaticity. In addition, a 4-dimensional one-hot vector may also be defined to indicate chemical bond types in a molecule graph, namely, a single bond, a double bond, a triple bond, and no chemical bond. The one-hot vector is a vector with only one element being 1 and remaining elements being 0. Each atom and each chemical bond have a label $s \in \{0,1\}$, indicating whether the atom or the chemical bond is an constituent involved when the product molecule is conversed into synthons.

For example, a message passing neural network (MPNN) at an L layer may be first used to encode each atom in a product molecule $G$, to obtain a feature vector of each atom in the product molecule. The MPNN is a supervised learning framework that may be applied to graphs. Further, a multilayer perceptron (MLP) may be used to encode each chemical bond in the product molecule $G$, to obtain a feature vector of each chemical bond. The MLP is a feedforward artificial neural network model. The MLP may map a plurality of inputted data sets into a single outputted data set.

For example, a feature vector $h_v$ of an atom and a feature vector $h_{v,u}$ of a chemical bond may be calculated in the following formulas:

$$h_v^L = MPNN\left(G, x_v, \{x_{v,u}\}_{u \in \mathcal{N}(v)}\right);$$

and

$$h_{v,u} = MLP_{bond}(h_v^L || h_u^L), u \in \mathcal{N}(v).$$

$MPNN(\cdot)$ indicates the MPNN, G indicates the graph structure of the product molecule, L indicates a quantity of layers of $MPNN(\cdot)$, $h_v^L$ indicates a feature vector of an atom $v$ outputted by an $L^{th}$ layer of $MPNN(\cdot)$, $x_v$ indicates a feature vector of the atom $v$ before encoding, $x_{v,u}$ indicates a feature vector of a chemical bond between the atom $v$ and an atom $u$ before encoding, $\mathcal{N}(v)$ indicates a set of neighbor nodes of the atom $v$, $h_{v,u}$ indicates a feature vector of the chemical bond between the atom $v$ and the atom $u$ after encoding, $MLP_{bond}(\cdot)$ indicates the MLP, $||$ indicates a splicing operation, and $h_u^L$ indicates a feature vector of the atom $u$ outputted by the $L^{th}$ layer of $MPNN(\cdot)$.

Further, for ease of a subsequent task, $h_{v,u}$ may be indicated in a self-loop form, that is:

$$h_{v,v} = MLP_{bond}(h_v^L || h_v^L).$$

Still further, to simplify calculations, an atom and a chemical bond are indicated in a same form, that is:

$$e_i \in \{h_{v,u}\}_{u \in \mathcal{N}(v) \cup v}.$$

i is a label of the atom or a label of the chemical bond.

For example, the label of the atom may be an index of the atom, and the label of the chemical bond may be an index of the chemical bond.

After a feature vector of the atom is obtained, a global attention pooling function may be used to calculate feature vectors of all atoms to obtain the feature vector $h_G$ of the product molecule. A feature vector of a synthon may be required, and has an obtaining manner same as $h_G$. A global attention pooling function may also be used to calculate feature vectors of all atoms included in the synthon to obtain a feature vector $h_{syn}$ of the synthon.

Certainly, in other alternative embodiments, in a process of encoding the product molecule or the synthon, a contrastive learning strategy may also be introduced, such as masking a graph structure or a feature of a molecule graph, that is, reactant molecule prediction performance may be improved by extending a feature dimension.

S220: Predict a conversion path between the product molecule and a plurality of reactant molecules by inputting the feature of the product molecule into a reverse reaction prediction model, the conversion path including an editing sequence and a synthon completion sequence.

For example, the editing sequence is a sequence formed by editing actions, and the synthon completion sequence is a sequence formed by synthon completion actions.

In other words, a prediction task of the reverse reaction prediction model is defined as a reactant molecule generation task. To be specific, the prediction task of the reverse reaction prediction model is as follows. A conversion path that describes a conversion path from a product molecule graph to a reactant molecule graph is predicted, where the conversion path is defined through an editing action for a product molecule and a synthon completion action for a synthon. In other words, the conversion path is constructed through an editing sequence formed by editing actions and a synthon completion sequence formed by synthon completion actions. In this way, a conversion path that describes a conversion path from the product molecule graph and the reactant molecule graph may be predefined for each product molecule. The synthon completion action may also be referred to as an *AddingMotif* action, that is, the synthon completion sequence may also be referred to as an *AddingMotif* sequence.

Alternatively, the conversion path includes the editing sequence and the synthon completion sequence. The editing sequence is configured for describing changes of chemical bonds and atoms from the product molecule to synthons. The synthon completion sequence is configured for describing a process of using motifs to complete the synthons. For the editing sequence, an editing action is introduced to indicate each change from the product molecule to the synthons. For the synthon completion sequence, a synthon completion action is introduced to describe each completion operation in the process of using the motifs to complete the synthons.

For example, the editing action is an action for editing an atom or a chemical bond in the product molecule in a process of conversing the product molecule into a plurality of synthons in the product molecule.

For example, the synthon completion action is an action for adding a motif in a process from the plurality of synthons to a plurality of reactant molecules.

For example, the editing sequence may further include an editing complete action. For example, the conversion path sequentially includes at least one editing action, the editing complete action, and at least one synthon completion action. The editing complete action is configured for connecting or distinguishing the at least one editing action and the at least one synthon completion action. In other words, the editing complete action is configured for triggering the reverse reaction prediction model to initiate a synthon completion task. In embodiments of this application, the editing complete action is innovatively introduced, to connect the at least one editing action to the at least one synthon completion action, to construct the conversion path.

For example, the editing sequence may further include a start action. For example, the conversion path sequentially includes the start action, at least one editing action, an editing complete action, and at least one synthon completion action. The start action is configured for triggering the reverse reaction prediction model to initiate a synthon prediction task or configured for triggering the reverse reaction prediction model to initiate a reactant molecule prediction task.

Certainly, in other alternative embodiments, the editing complete action may also be used as an action in the synthon completion sequence. This is not specifically limited in this application.

The reverse reaction prediction model in this application may be any deep learning or ML model for recognition. A specific type thereof is not specifically limited in embodiments of this application.

For example, the reverse reaction prediction model includes, but not limited to: a conventional learning model, an integrated learning model, or a deep learning model. In one embodiment, the conventional learning model includes, but not limited to: a tree model (a regression tree) or a logistic regression (LR) model; the integrated learning model includes, but not limited to: an improvement model (XGBoost) for a gradient boosting algorithm or a random forest model; and the deep learning model includes, but not limited to: a neural network, a dynamic Bayesian network (DBN), or a stacked auto-encoder network (SAE) model. Certainly, in other embodiments of this application, a model of another ML type may alternatively be used.

For example, the model may be trained by using a batch size.

The batch size is a quantity of samples selected for one training. A value of the batch size determines smoothness of a gradient between time required to complete each epoch and each iteration in a deep learning training process. For a training set with a size of N, if a batch size sampling method in each epoch uses that a most conventional method, that is, N samples each are sampled once, and a batch size is b, a quantity of iterations required in each epoch is N/b. Therefore, the time required to complete each epoch roughly also increases with the quantity of iterations. If the value of the batch size is too small, it takes a lot of time, and the gradient will oscillate seriously, which is not conducive to convergence. If the value of the batch size is too large, there is no change in a gradient direction for different batch sizes, and it is easy to fall into a local minimum value. The value of the batch size is not limited in embodiments of this application, for example, a suitable batch size may be determined based on an actual requirement or scenario. Certainly, in other alternative embodiments, for a database with a small quantity of samples, not using a batch size is also feasible and works well. However, for a large database, inputting all data into a network at once definitely causes an explosion of a memory. In this case, a batch size may be used for network training.

To better understand embodiments of this application, content related to this application is described.

Neural network (NN).

The NN is a computing model formed by a plurality of neuron nodes connected to each other, where a connection between the nodes indicate a weighted value from an input signal to an output signal, which is referred to as a weight. Each node performs weighted summation (SUM) on different input signals, and performs an output through a specified activation function (f).

For example, the NN includes, but not limited to: a deep neural network (DNN), a convolutional neural network (CNN), a recurrent neural network (RNN), and the like.

Convolutional neural network (CNN):

The CNN, as a feedforward neural network that includes convolution calculation and has a deep structure, is one of representative algorithms of deep learning. Because the CNN can perform shift-invariant classification, the CNN is also referred to as a shift-invariant artificial neural network (SIANN).

Recurrent neural network (RNN):

The RNN is a neural network that models sequence data, and has achieved remarkable results in the field of natural language processing, such as machine translation, and speech recognition. A specific expression is that the network memorizes information from past moments, and applies the information from past moments to calculation of a current output. To be specific, nodes between hidden layers are connected, and inputs of the hidden layers not only include an output of an input layer but also include outputs of hidden layers at a previous moment. A commonly used RNN includes structures such as a long short-term memory (LSTM) network and a gated recurrent unit (GRU).

S230: Edit in simulation, for each editing action in the editing sequence, an constituent indicated by the respective editing action based on a target state indicated by the respective editing action, to obtain a plurality of synthons corresponding to the product molecule, the constituent being an atom or a chemical bond in the product molecule.

For example, for each editing action in the editing sequence, the constituent indicated by an editing action is edited based on an order of the editing action in the editing sequence and the target state indicated by the editing action in the editing sequence, to obtain the plurality of synthons corresponding to the product molecule.

In some embodiments, if the constituent indicated by the respective editing action is the atom, the target state indicated by the respective editing action is changing a quantity of charges on the atom or changing a quantity of hydrogen atoms on the atom; or if the constituent indicated by the respective editing action is the chemical bond, the target state indicated by the respective editing action is any one of the following: the chemical bond is added, the chemical bond is deleted, and the chemical bond is changed in type.

For example, the target state indicated by the respective editing action is related to the constituent indicated by the respective editing action.

For example, if the constituent indicated by the respective editing action is the chemical bond, the target state indicated by the respective editing action includes, but not limited to the following states: adding the chemical bond, deleting the chemical bond, or changing the type of the chemical bond. For another example, if the constituent indicated by the respective editing action is the atom, the target state indicated by the respective editing action includes, but not limited to the following states: changing the quantity of charges on the atom or changing the quantity of hydrogen atoms.

In some embodiments, the editing action is represented through the following labels: an action label configured for indicating editing, a label configured for indicating the object, and a label configured for indicating the target state.

For example, the editing action in the editing sequence may be defined as an editing triplet, namely, $(\pi 1, o, \tau)$, where $\pi 1$ indicates that an action predicted by the reverse reaction prediction model is an editing action, o indicates a label of an constituent corresponding to the editing action predicted by the reverse reaction prediction model, and $\tau$ indicates a label of a target state corresponding to the editing action predicted by the reverse reaction prediction model. For example, assuming that an editing triplet is $(\pi 1, b, none)$, $\pi 1$ indicates an action predicted by the reverse reaction prediction model is an editing action, b indicates that an constituent corresponding to the editing action is a chemical bond whose label is b, and none indicates that a target state corresponding to the editing action is deleting the chemical bond whose label is b.

Certainly, in other alternative embodiments, the editing action may be defined as a 2-tuple or a value in another form. For example, when the editing action is defined as the 2-tuple, the editing action may be specifically defined as the label configured for indicating the constituent and the label configured for indicating the target state.

S240: performing, for each of the plurality of synthons, at least one synthon completion action corresponding to the respective synthon, to add to the respective synthon, the motif indicated by the at least one corresponding synthon completion action according to the corresponding interface atom, to obtain a predicted reactant molecule corresponding to a respective one of the plurality of synthons, so as to finally obtain a plurality of predicted reactant molecules respectively corresponding to the plurality of synthons, the motif including a plurality of atoms or atomic edges for connecting the plurality of atoms.

For example, the atomic edge may represent acting force between two or more atoms connected by the atomic edge. The acting force is configured for binding the atoms connected by the atomic edge.

For example, the atomic edge is a chemical bond configured for connecting different atoms in the plurality of atoms. The atomic edge may be a chemical bond, which includes, but not limited to: an ionic bond, a covalent bond, or a metallic bond.

For example, the synthon is a molecular fragment obtained by disconnecting a chemical bond in a product molecule graph.

For example, the motif is a sub-graph of a reactant. For example, the motif may be a sub-graph on a reactant corresponding to the synthon. The reactant corresponding to the synthon may include a molecule or a reactant including the synthon.

The motif may include sub-graphs obtained in the following manners:

1. Edges that differ between the synthon and the reactant corresponding to the synthon are disconnected, to obtain in a series of sub-graphs. Interface atoms corresponding to attached atoms on the synthon are reserved on the sub-graphs.

2. If there are two atoms that are connected to each other and that respectively belong to two rings on a sub-graph, a chemical bond connected between the two atoms is disconnected, to obtain two smaller sub-graphs.

It is to be understood that, the ring in embodiments of this application may be a single ring, in other words, a molecule has only one ring. Correspondingly, if there are two atoms that are connected to each other and that respectively belong to two single rings on a sub-graph, a chemical bond connected between the two atoms is disconnected, to obtain two smaller sub-graphs. In addition, the ring in embodiments of this application may be a cycloalkane, and the cycloalkane may be classified based on a quantity of carbon atoms on the ring. For example, the cycloalkane is referred to as a small ring when the quantity of carbon atoms on the ring is 3 to 4, the cycloalkane is referred to as an ordinary ring when the quantity of carbon atoms on the ring is 5 to 6, the cycloalkane is referred to as a middle ring when the quantity of carbon atoms on the ring is 7 to 12, and the cycloalkane is referred to as a large ring when the quantity of carbon atoms on the ring is greater than 12.

3. For two atoms connected to each other on a sub-graph, if one atom belongs to a ring and a degree of the other atom is greater than 1, a chemical bond connected between the two atoms is disconnected, to obtain two smaller sub-graphs.

For example, the reverse reaction prediction model predicts the synthon completion sequence based on a first order of the plurality of synthons, and a traversing order of the plurality of synthons is a second order when the motif is added for each synthon in the plurality of synthons. In this case, the at least one synthon completion action corresponding to each synthon in the synthon completion sequence may be determined based on the first order and the second order, to add the motif indicated by each synthon completion action based on the interface atom indicated by each synthon completion action in the at least one synthon completion action. The first order and the second order may be the same or different. For example, the at least one synthon completion action corresponding to each synthon in the synthon completion sequence may be determined based on the first order, the second order, and a quantity of the at least one synthon completion action corresponding to each synthon, to add the motif indicated by each synthon completion action based on the interface atom indicated by each synthon completion action in the at least one synthon completion action.

It is assumed that the first order is equal to the second order, and the quantity of the at least one synthon completion action corresponding to each synthon is a preset value. In this case, based on the traversing order of the plurality of synthons used when the reverse reaction prediction model predicts the synthon completion sequence, sequentially for each synthon in the plurality of synthons, the motif indicated by each synthon completion action may be added based on the interface atom indicated by each synthon completion action in the preset quantity of synthon completion actions corresponding to each synthon. For example, it is assumed that the plurality of synthons include a synthon 1 and a synthon 2, a first synthon completion action in the synthon completion sequence is a synthon completion action for the synthon 1, and remaining synthon completion actions other than the first synthon completion action in the synthon completion sequence are synthon completion actions for the synthon 2. In this case, a motif indicated by the first synthon completion action may be added based on an interface atom indicated by the first synthon completion action, to obtain a plurality of reactant molecules corresponding to the synthon 1. Further, motifs indicated by the remaining synthon completion actions may be sequentially added based on interface atoms indicated by the remaining synthon completion actions, to obtain a plurality of reactant molecules corresponding to the synthon 2.

For example, the interface atom indicated by each synthon completion action is an atom that is on the motif indicated by each synthon completion action and that is used as a connection node when synthon completion is performed by using the motif indicated by each synthon completion action.

For example, when a motif indicated by a synthon completion action is added, an interface atom indicated by the synthon completion action and an attached atom for the synthon completion action may be used as a connection node, to obtain the plurality of reactant molecules corresponding to the product molecule. The interface atom indicated by the synthon completion action and the attached atom for the synthon completion action are a same atom in the reactant molecules.

For example, attached atoms in this embodiment of this application include an atom selected as an constituent and atoms at both ends of a chemical bond selected as an object.

In some embodiments, the synthon completion action is represented through the following labels: an action label configured for indicating to perform synthon completion, a label configured for indicating the motif, and a label configured for indicating the interface atom.

For example, the synthon completion action in the synthon completion sequence may be defined as a synthon completion triplet, namely, $(\pi3, z, q)$, where $\pi3$ indicates an action predicted by the reverse reaction prediction model is a synthon completion action, z indicates a label of a motif indicated by the synthon completion action predicted by the reverse reaction prediction model, and q indicates a label of an interface atom corresponding to the synthon completion action predicted by the reverse reaction prediction model. For example, a synthon completion triplet is $(\pi3, z1, q1)$, where $\pi3$ indicates an action predicted by the reverse reaction prediction model is a synthon completion action; z1 indicates a label of a motif indicated by the synthon completion action predicted by the reverse reaction prediction model, namely, a motif whose label is z1; and q1 indicates a label of an interface atom corresponding to the synthon completion action predicted by the reverse reaction prediction model, namely, an atom whose label is q1 in the motif whose label is z1. Based on this, based on $(\pi3, z1, q1)$, the atom whose label is q1 in the motif whose label is z1 may be used as an interface atom, and synthon completion may be performed by using the z1 motif.

Certainly, in other alternative embodiments, the synthon completion action may be defined as a 2-tuple or a value in another form. For example, when the synthon completion action is defined as the 2-tuple, the synthon completion action may be specifically defined as the label configured for indicating the motif and the label configured for indicating the interface atom.

By introducing a reverse reaction prediction model configured to predict a conversion path between a product molecule and a plurality of reactant molecules, a synthon prediction task and a synthon completion prediction task can be merged. In other words, the reverse reaction prediction model introduced in the embodiments of this application can learn a potential relationship between two sub-tasks: synthon prediction and synthon completion. In this way, generalization performance of the model is greatly improved, reactant molecule prediction complexity can be reduced, and generalization performance of reactant molecule prediction can be improved. In addition, by introducing a motif and designing the motif as a structure including a plurality of atoms or atomic edges for connecting the plurality of atoms, a short and accurate conversion path can be reasonably constructed. In this way, an excessively long length of a synthon completion sequence is avoided, reactant molecule prediction difficulty is reduced, and reactant molecule prediction accuracy is improved, so that reactant molecule prediction performance can be improved.

In addition, by improving the reactant molecule prediction performance, the following technical effects can also be obtained:

1. Synthetic paths may be planned for designed drugs or new material molecules, thereby improving research efficiency of the drugs or the new material molecules.

2. Some potential scientific laws may be revealed, thereby providing new scientific knowledge.

3. More accurate synthesis route plans than those of professional researchers may be provided. In this way, reliable reactant molecules can be predicted in the absence of reaction templates, and reaction types that have not been clearly defined by the professional researchers can also be predicted, thereby greatly improving research and development efficiency of new drugs and new materials.

In some embodiments, S220 may include:
obtaining an input feature of a $t^{th}$ action based on a $(t-1)^{th}$ action obtained through prediction of the reverse reaction prediction model, where t is an integer greater than 1; and predicting the $t^{th}$ action based on the input feature corresponding to the $t^{th}$ action and a hidden feature corresponding to the $t^{th}$ action, and obtaining the conversion path when an action obtained through prediction of the reverse reaction prediction model is a synthon completion action and all attached atoms on the plurality of synthons and all attached atoms on motifs added for the plurality of synthons have been traversed, where the hidden feature of the $t^{th}$ action is related to an action predicted by the reverse reaction prediction model before the $t^{th}$ action.

For example, attached atoms include an atom selected as an constituent and atoms at both ends of a chemical bond selected as an object.

For example, the reverse reaction prediction model may be the RNN. Based on this, the $(t-1)^{th}$ action may correspond to a moment t-1, and the $t^{th}$ action may correspond to a moment t. In other words, an input feature of the RNN at the moment t is obtained based on an action obtained through prediction of the RNN at the moment t-1, an action of the RNN at the moment t is predicted based on the input feature of the RNN at the moment t and a hidden feature of the RNN at the moment t, and the conversion path is obtained until the action obtained through prediction of the RNN is a synthon completion action and all the attached atoms on the plurality of synthons and all the attached atoms on motifs added for the plurality of synthons have been traversed.

The MLP and the CNN have a characteristic, to be specific, assuming that an input is an independent unit without context, for example, an input is a picture, a network recognizes whether the input is a dog or a cat. However, for some serialized inputs with clear contextual features, for example, playback content of a next frame in a prediction video, it is clear that such an output necessarily relies on a previous input, which means that the network necessarily has a memory capability. The RNN may just give the network the memory capability.

Certainly, in other alternative embodiments, the reverse reaction prediction model may also be another model. This is not specifically limited in this application.

For example, when predicting the $t^{th}$ action, the reverse reaction prediction model may obtain an output feature $\boldsymbol{u}_t$ based on the input feature corresponding to the $t^{th}$ action and the hidden feature corresponding to the $t^{th}$ action, and splice the output feature $\boldsymbol{u}_t$ and a feature $\boldsymbol{h}_G$ of the product molecule, to obtain a feature $\psi_t$ configured for recognizing the $t^{th}$ action.

For example, the output feature $\boldsymbol{u}_t$ and the feature $\psi_t$ configured for recognizing the $t^{th}$ action may be obtained in the following manner:

$$\boldsymbol{u}_t = GRU(input_{t-1}, hidden_{t-1}), where\ input_0 = \mathbf{0},\ and\ hidden_0 = \sigma_G(\boldsymbol{h}_G);$$

and

$$\psi_t = \boldsymbol{h}_G || \boldsymbol{u}_t.$$

$GRU(\cdot)$ is a gated recurrent unit (GRU); $input_{t-1}$ and $hidden_{t-1}$ are respectively an input feature used by the GRU to predict the $t^{th}$ action (namely, a feature of an intermediate molecular fragment after editing based on the $(t-1)^{th}$ action) and a hidden state passed by a node configured to predict the $t^{th}$ action, and initial values thereof are respectively a vector $\mathbf{0}$ and a feature of the product molecule; and $\sigma_G(\cdot)$ is an embedding function of the feature of the product molecule.

For example, the reverse reaction prediction model may recognize the $t^{th}$ action by using the following formula:

$$\hat{\pi}_t = softmax\big(MLP_{act}(\psi_t)\big).$$

$softmax(\cdot)$ is a classification function, $MLP_{bond}(\cdot)$ indicates the MLP, and $\psi_t$ indicates the feature configured for recognizing the $t^{th}$ action.

It is to be understood that, in embodiments of this application, the output feature $\boldsymbol{u}_t$ and the feature $\boldsymbol{h}_G$ of the product molecule is spliced, that is, the feature of the product molecule and the output feature of the RNN is spliced for subsequent action prediction, which can integrate global topology information into an action prediction process, to improve action prediction accuracy.

In some embodiments, the conversion path may be determined in the following manner:

obtaining, based on a beam search manner of a hyperparameter k, k first prediction results with highest scores from prediction results of the $(t-1)^{th}$ action; determining k first input features corresponding to the $t^{th}$ action based on the k first prediction results; predicting the $t^{th}$ action based on each first input feature in the k first input features and the hidden feature corresponding to the $t^{th}$ action, and obtaining, based on the beam search manner of the hyperparameter k, k second prediction results with highest scores from prediction results obtained through prediction; determining k second input features corresponding to a $(t+1)^{th}$ action based on the k second prediction results; and predicting the

$(t+1)^{th}$ action based on each second input feature in the k second input features and a hidden feature corresponding to the $(t+1)^{th}$ action, and obtaining the conversion path when an action obtained through prediction of the reverse reaction prediction model is a synthon completion action and all attached atoms on the plurality of synthons and all attached atoms on motifs added for the plurality of synthons have been traversed.

For example, when predicting the $t^{th}$ action based on each first input feature in the k first input features and the hidden feature corresponding to the $t^{th}$ action and obtaining 2k prediction results, a prediction apparatus may obtain, based on the beam search manner of the hyperparameter k, k second prediction results with highest scores from the 2k prediction results obtained through prediction, and determine the k second prediction results as k second input features corresponding to the $(t+1)^{th}$ action.

For example, when the k first prediction results with highest scores from the prediction results of the $(t-1)^{th}$ action are obtained based on the beam search manner of the hyperparameter k, the prediction results of the $(t-1)^{th}$ action may be sorted based on scores, and then k prediction results with highest scores in the prediction results of the $(t-1)^{th}$ action may be used as the k first prediction results. For example, when the prediction results of the $(t-1)^{th}$ action may be sorted based on the scores, a cumulative sum of scores of all predicted prediction results on a path where each prediction result is located may be first calculated, to obtain the cumulative sum of scores corresponding to each prediction result, and then k prediction results with highest cumulative sums of scores in the prediction results of the $(t-1)^{th}$ action may be used as the k first prediction results.

For example, beam search of the hyperparameter k is configured to select a plurality of alternative solutions for an input sequence at each time step based on conditional probabilities. A quantity of the plurality of alternative solutions depends on a hyperparameter k referred to as a beam width. At each moment, the beam search selects k best alternative solutions with highest probabilities as most likely choices at the current moment. In other words, at each moment, a best result k with a highest score is selected as an input of a next moment based on a log-likelihood score function. In other words, such a process may be described as construction of a search tree, where a leaf node with a highest score is expanded with sub-nodes thereof, while other leaf nodes are deleted.

In some embodiments, if the $(t-1)^{th}$ action is the editing action, the input feature of the $t^{th}$ action is determined based on a feature of a sub-graph obtained through editing by using the $(t-1)^{th}$ action; an constituent and a target state that are indicated by the $t^{th}$ action by using the reverse reaction prediction model is predicted based on the input feature of the $t^{th}$ action and the hidden feature of the $t^{th}$ action; and the editing sequence is obtained until an action obtained through prediction of the reverse reaction prediction model is a final editing action in the editing sequence.

For example, the final editing action is an editing complete action.

In other words, if the $(t-1)^{th}$ action is the editing action, the input feature of the $t^{th}$ action is determined based on the feature of the sub-graph obtained through editing by using the $(t-1)^{th}$ action; the constituent and the target state that are indicated by the $t^{th}$ action by using the reverse reaction prediction model is predicted based on the input feature of the $t^{th}$ action and the hidden feature of the $t^{th}$ action; and the editing sequence is obtained until the action obtained through prediction of the reverse reaction prediction model is the editing complete action.

For example, t is an integer greater than 1 or greater than 2.

For example, when the $(t-1)^{th}$ action is a first editing action, the $(t-1)^{th}$ action is a start action, and in this case, the feature of the sub-graph obtained through editing by using the $(t-1)^{th}$ action is a feature of the product molecule.

For example, when predicting the $t^{th}$ action, the reverse reaction prediction model may decode an intermediate molecular fragment after editing based on the $(t-1)^{th}$ action, to obtain a feature of the intermediate molecular fragment after editing based on the $(t-1)^{th}$ action.

For example, if the $t^{th}$ action is the editing action, the reverse reaction prediction model may allocate a score $\hat{S}_i$ based on each chemical bond and each atom, and predict the constituent corresponding to the $t^{th}$ action.

For example, if the $t^{th}$ action is the editing action, the reverse reaction prediction model may first allocate a score $\hat{S}_i$ to each chemical bond and each atom when predicting the constituent corresponding to the $t^{th}$ action, where the score indicates a probability that the chemical bond or the atom is considered as the constituent in the $(t-1)^{th}$ action; and may predict the constituent corresponding to the $t^{th}$ action based on the score $\hat{S}_i$ allocated to each chemical bond and each atom.

For example, the reverse reaction prediction model may obtain the score $\hat{S}_i$ allocated to each chemical bond and each atom by using the following formula:

$$\hat{s}_i = sigmoid\left(MLP_{target}(\psi_t || \sigma_e(e_i))\right).$$

$\hat{S}_i$ indicates a score of an $i^{th}$ chemical bond or atom, sigmoid($\cdot$) is a logistic regression function, $MLP_{target}(\cdot)$ indicates a feature that is outputted by the MLP and that is configured for determining the score of the $i^{th}$ chemical bond

or atom, $\psi_t$ indicates a feature configured for recognizing the $t^{th}$ action, $\sigma_e(\cdot)$ indicates an embedding function of a feature of an atom or a chemical bond, and $e_i$ indicates the $i^{th}$ chemical bond or atom.

Then, the reverse reaction prediction model predicts a target state $\hat{r}_b$ for the constituent corresponding to the $t^{th}$ action.

For example, the reverse reaction prediction model may predict the target state $\hat{r}_b$ for the constituent corresponding to the $t^{th}$ action by using the following formula:

$$\hat{r}_b = softmax\left(MLP_{type}\left(\psi_t || \sigma_e\left(e_{\mathrm{argmax}_i(\hat{s}_i)}\right)\right)\right).$$

$\hat{r}_b$ indicates the target state of the constituent obtained through prediction, for example, an edited chemical bond type, $softmax(\cdot)$ is a classification function, $MLP_{type}(\cdot)$ indicates a feature that is outputted by the MLP and that is configured for determining a chemical bond type, $\psi_t$ indicates the feature configured for recognizing the $t^{th}$ action, $\sigma_e(\cdot)$ indicates the embedding function of the feature of the atom or the chemical bond, $\mathrm{argmax}(\cdot)$ indicates to look up an atom or a chemical bond with a highest score, and $e_{\mathrm{argmax}_i(\hat{s}_i)}$ indicates a feature of the atom or the chemical bond with the highest score.

For example, the reverse reaction prediction model applies the constituent corresponding to the $t^{th}$ action and the target state corresponding to the $t^{th}$ action that are obtained through prediction to an intermediate molecular fragment before editing corresponding to the $t^{th}$ action, to obtain an intermediate molecular fragment after editing corresponding to the $t^{th}$ action, and calculates a feature $h_t^{syn}$ of the intermediate molecular fragment after editing corresponding to the $t^{th}$ action by using $MPNN(\cdot)$. Then, an input feature $input_t$ corresponding to the $(t+1)^{th}$ action is obtained based on the obtained feature $h_t^{syn}$ of the intermediate molecular fragment after editing corresponding to the $t^{th}$ action, the constituent corresponding to the $t^{th}$ action and the target state corresponding to the $t^{th}$ action.

For example, the input feature $input_t$ corresponding to the $(t+1)^{th}$ action may be obtained by using the following formula:

$$input_t = h_t^{syn} + \sigma_e\left(e_{\mathrm{argmax}_i(\hat{s}_i)}\right) + \sigma_b(\hat{r}_b).$$

$h_t^{syn}$ indicates the obtained feature of the intermediate molecular fragment after editing corresponding to the $t^{th}$ action, $\sigma_e(\cdot)$ indicates the embedding function of the feature of the atom or the chemical bond, $\mathrm{argmax}(\cdot)$ indicates to look up the atom or the chemical bond with the highest score, $e_{\mathrm{argmax}_i(\hat{s}_i)}$ indicates the feature of the atom or the chemical bond with the highest score, $\hat{s}_i$ indicates a score of the $i^{th}$ chemical bond or atom, $\hat{r}_b$ indicates the target state of the constituent obtained through prediction, and $\sigma_b(\cdot)$ indicates an embedding function of $\hat{r}_b$.

In some embodiments, if the $(t-1)^{th}$ action is a final editing action or the synthon completion action in the editing sequence, the input feature of the $t^{th}$ action is determined based on a feature of a sub-graph obtained through editing by using the $(t-1)^{th}$ action and a feature of an attached atom corresponding to the $(t-1)^{th}$ action; a motif and an interface atom that are indicated by the $t^{th}$ action are predicted based on the input feature of the $t^{th}$ action and the hidden feature of the $t^{th}$ action; and the synthon completion sequence is obtained until an action obtained through prediction of the reverse reaction prediction model is a synthon completion action and all attached atoms on the plurality of synthons and all attached atoms on motifs added for the plurality of synthons have been traversed.

For example, the final editing action is an editing complete action.

In other words, if the $(t-1)^{th}$ action is the editing complete action or the synthon completion action, the input feature of the $t^{th}$ action is determined based on the feature of the sub-graph obtained through editing by using the $(t-1)^{th}$ action and the feature of the attached atom corresponding to the $(t-1)^{th}$ action; the motif and the interface atom that are indicated by the $t^{th}$ action are predicted based on the input feature of the $t^{th}$ action and the hidden feature of the $t^{th}$ action; and the synthon completion sequence is obtained until the action obtained through prediction of the reverse reaction prediction model is the synthon completion action and all the attached atoms on the plurality of synthons and all the attached atoms on the motifs added for the plurality of synthons have been traversed.

For example, if the action obtained through prediction of the reverse reaction prediction model is the final editing action, it indicates that a synthon prediction stage ends, and a reactant prediction process enters a synthon completion stage. In this case, all the attached atoms are sorted based on label orders thereof in the product molecule, for ease of synthon completion.

For example, if the action obtained through prediction of the reverse reaction prediction model is the final editing action, the input feature *input$_t$* corresponding to the (t+1)$^{th}$ action if may be determined by using the following formula:

$$input_t = \boldsymbol{h}_{syn} + \sigma_{atom}(a_t), where\ a_t \in \{a\}.$$

$\boldsymbol{h}_t^{syn}$ indicates the obtained feature of the intermediate molecular fragment after editing corresponding to the t$^{th}$ action, $\sigma_{atom}(\cdot)$ indicates an embedding function of a feature of an attached atom, and $a_t$ indicates the attached atom corresponding to the t$^{th}$ action.

For example, in the synthon completion stage, the reverse reaction prediction model sequentially traverses all the attached atoms on the synthons and all the attached atoms on the added motifs, and allocates a motif to each attached atom. Motif prediction may be regarded as a multi-classification task in a pre-stored dictionary *Z*. After a motif $\hat{z}$ is obtained through motif prediction, an interface atom $\hat{q}$ corresponding to the attached atom $a_t$ on the motif $\hat{z}$ may be further determined.

For example, the reverse reaction prediction model may predict the motif $\hat{z}$ corresponding to the t$^{th}$ action by using the following formula:

$$\hat{z} = softmax\left(MLP_{motif}(\boldsymbol{\psi}_t)\right).$$

$\hat{z}$ indicates the motif corresponding to the t$^{th}$ action, *softmax*($\cdot$) is the classification function, *MLP$_{motif}$*($\cdot$) indicates a feature that is outputted by the MLP and that is configured for predicting the motif corresponding to the t$^{th}$ action, and $\psi_t$ indicates the feature configured for recognizing the t$^{th}$ action.

For example, the reverse reaction prediction model may predict the interface atom $\hat{q}$ corresponding to the attached atom $a_t$ on the motif $\hat{z}$ by using the following formula:

$$\hat{q} = softmax\left(MLP_{interface}(\boldsymbol{\psi}_t||\sigma_z(\hat{z}))\right).$$

$q$ indicates the interface atom corresponding to the attached atom $a_t$ on the motif $\hat{z}$ corresponding to the t$^{th}$ action, *softmax*($\cdot$) is the classification function, *MLP$_{interface}$*($\cdot$) indicates a feature that is outputted by the MLP and that is configured for predicting $\hat{q}$, $\psi_t$ indicates the feature configured for recognizing the t$^{th}$ action, $\sigma_z(\cdot)$ indicates an embedding function of the feature of the motif $\hat{z}$, and $\hat{z}$ indicates the motif corresponding to the t$^{th}$ action.

If the (t-1)$^{th}$ action is the final editing action or the synthon completion action, and an interface atom of a motif corresponding to the (t-1)$^{th}$ action includes only one interface atom, the input feature of the t$^{th}$ action is determined based on the feature of the sub-graph obtained through editing by using the (t-1)$^{th}$ action and a feature of the attached atom corresponding to the (t-1)$^{th}$ action.

For example, if the motif $\hat{z}$ obtained through prediction includes only one interface atom, the input feature *input$_t$* corresponding to the (t+1)$^{th}$ action may be determined by using the following formula:

$$input_t = \boldsymbol{h}_{syn} + \sigma_{atom}(a_t), where\ a_t \in \{a\}.$$

$\boldsymbol{h}_t^{syn}$ indicates the obtained feature of the intermediate molecular fragment after editing corresponding to the t$^{th}$ action, $\sigma_{atom}(\cdot)$ indicates the embedding function of the feature of the attached atom, and $a_t$ indicates the attached atom corresponding to the t$^{th}$ action.

If the (t-1)$^{th}$ action is the final editing action or the synthon completion action, and an interface atom of a motif corresponding to the (t-1)$^{th}$ action includes only a plurality of interface atoms, the input feature of the t$^{th}$ action is determined based on the feature of the sub-graph obtained through editing by using the (t-1)$^{th}$ action, a feature of the motif corresponding to the (t-1)$^{th}$ action, and features of the attached atoms corresponding to the (t-1)$^{th}$ action.

For example, if the motif $\hat{z}$ obtained through prediction includes only the plurality of interface atoms, the input feature *input$_t$* corresponding to the (t+1)$^{th}$ action may be determined by using the following formula:

$$input_t = \boldsymbol{h}_{syn} + \sigma_z(\hat{z}) + \sigma_{atom}(a_t).$$

$\boldsymbol{h}_t^{syn}$ indicates the obtained feature of the intermediate molecular fragment after editing corresponding to the $t^{th}$ action, $\sigma_z(\cdot)$ indicates the embedding function of the feature of the motif $\hat{z}$, $\hat{z}$ indicates the motif corresponding to the $t^{th}$ action, $\sigma_{atom}(\cdot)$ indicates the embedding function of the feature of the attached atom, and $a_t$ indicates the attached atom corresponding to the $t^{th}$ action.

It can be learned through the foregoing solutions that, the conversion path may be obtained after the reverse reaction prediction model predicts the editing sequence and the synthon completion sequence, so that the conversion path may be acted on the product molecule graph to obtain the reactant molecule graph.

FIG. 3 shows an example of a conversion path according to an embodiment of this application.

As shown in FIG. 3, when a reverse reaction prediction model is used to perform reactant prediction on a product molecule shown in a left side of (a) of FIG. 3, a conversion path shown in (b) of FIG. 3 may be obtained based on a procedure shown in (c) of FIG. 3, to obtain two reactant molecules shown in a right side of (a) of FIG. 3. $\pi1$ indicates an action predicted by the reverse reaction prediction model is an editing action, $\pi2$ indicates an action predicted by the reverse reaction prediction model is an editing complete action, $\pi3$ indicates an action predicted by the reverse reaction prediction model is a synthon completion action, a1 to a3 indicate attached atoms, q1 to q4 indicate interface atoms, z1 to z3 indicate motifs, b indicates that an constituent corresponding to the editing action is a chemical bond whose label is b, and none indicates that a target state corresponding to the editing action is deleting the chemical bond whose label is b. An attached atom is an interface for adding a motif.

Specifically, when an input of the reverse reaction prediction model is a start action, a label of a predicted first editing action is a triplet ($\pi1$, b, none), and then a label of a second action obtained through prediction is $\pi2$ by using the triplet ($\pi$-1, b, none) as an input. Then, all attached atoms are sorted based on label orders thereof in the product molecule, 2-tuples ($\pi3$, a1), ($\pi3$, a3), and ($\pi3$, a3) are sequentially inputted, and labels of actions obtained through sequential prediction are a triplet ($\pi3$, z1, q1), a triplet ($\pi3$, z2, q2), and a triplet ($\pi3$, z3, q4). Based on this, an obtained conversion path may be defined as the path shown in (b) of FIG. 3: a triplet ($\pi1$, b, none), $\pi2$, a triplet ($\pi3$, z1, q1), a triplet ($\pi3$, z2, q2), and a triplet ($\pi3$, z3, q4). In this way, the conversion path is acted on the product molecule, so that the reactant molecules shown in the right side of (a) of FIG. 3 can be obtained.

In other words, in a reactant prediction process shown in FIG. 3, an editing sequence includes only one editing action, which is defined as the triplet ($\pi1$, b, none); and a synthon completion sequence includes three synthon completion actions, which are respectively defined as the triplet ($\pi3$, z1, q1), the triplet ($\pi3$, z2, q2), and the triplet ($\pi3$, z3, q4).

When a motif indicated by a synthon completion action is added, an interface atom indicated by the synthon completion action and an attached atom for the synthon completion action may be used as a connection node. The interface atom indicated by the synthon completion action and the attached atom for the synthon completion action are a same atom in the reactant molecules. For example, when z1 is added based on the triplet ($\pi3$, z1, q1), a1 and q1 are a same atom (namely, an atom N) in the reactant molecules; similarly, when z2 is added based on the triplet ($\pi3$, z2, q2), a2 and q2 are a same atom (namely, an atom O) in the reactant molecules; and after z2 is added, the interface atom q3 changes to the attached atom a3, and in this case, when z3 is added based on the triplet ($\pi3$, z3, q4), a3 and q4 are a same atom (namely, an atom C) in the reactant molecules.

It is to be understood that, FIG. 3 is an example of this application, and is not to be construed as a limitation to this application.

For example, in other alternative embodiments, the conversion path may alternatively include another quantity of editing actions or synthon completion actions, and even include another quantity of synthons or reactant molecules. This is not specifically limited in embodiments of this application.

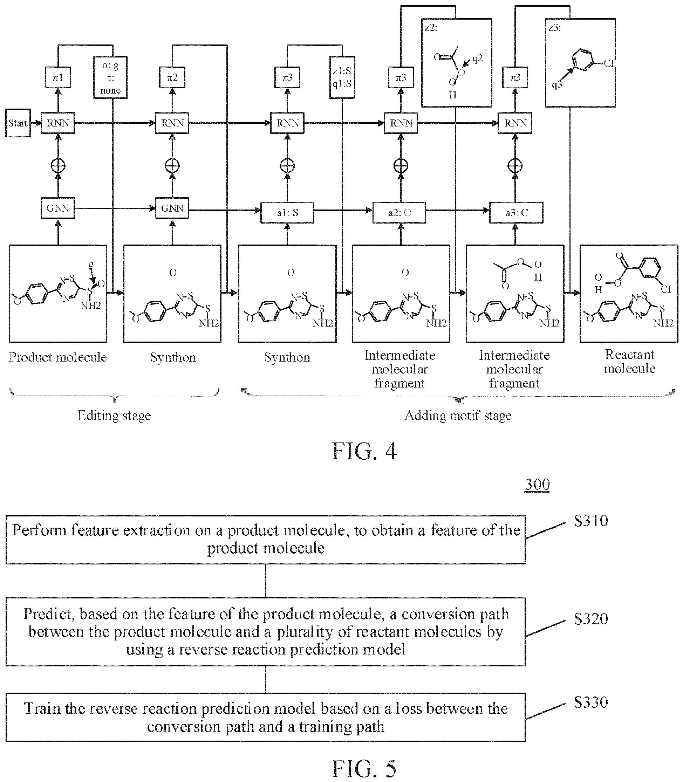

FIG. 4 is another schematic flowchart of a method for predicting a reactant molecule according to an embodiment of this application. $\pi1$ indicates an action predicted by a reverse reaction prediction model is an editing action, $\pi2$ indicates an action predicted by the reverse reaction prediction model is an editing complete action, $\pi3$ indicates an action predicted by the reverse reaction prediction model is a synthon completion action, a1 to a3 indicate attached atoms, q1 to q3 indicate interface atoms, z1 to z3 indicate motifs, g indicates that an constituent corresponding to the editing action is a chemical bond whose label is g, and none indicates that a target state corresponding to the editing action is deleting the chemical bond whose label is g. An attached atom is an interface for adding a motif.

Specifically, when an input of the reverse reaction prediction model is a start action, a label of a predicted first editing action is a triplet ($\pi1$, g, none), and then a label of a second action obtained through prediction is $\pi2$ by using the triplet ($\pi1$, g, none) as an input. Then, all attached atoms are sorted based on label orders thereof in a product molecule, 2-tuples ($\pi3$, a1), ($\pi3$, a3), and ($\pi3$, a3) are sequentially inputted, and labels of actions obtained through sequential prediction are a triplet ($\pi3$, z1, q1), a triplet ($\pi3$, z2, q2), and a triplet ($\pi3$, z3, q4). Based on this, an obtained conversion path may be defined as a path shown in FIG. 4: a triplet ($\pi 1$, g, none), $\pi 2$, a triplet ($\pi 3$, $z1$, $q1$), a triplet ($\pi 3$, $z2$, $q2$), and a triplet ($\pi 3$, $z3$, $q4$), where a1 to a3, q1 to q3, and z1 to z3 are shown in the figure In this way, the conversion path is acted on a product molecule, so that final reactant molecules can be obtained.

In a reactant prediction process shown in FIG. 4, an editing sequence includes only one editing action, which is defined as the triplet ($\pi 1$, g, none); and a synthon completion sequence includes three synthon completion actions, which are respectively defined as the triplet ($\pi 3$, $z1$, q1), the triplet ($\pi 3$, $z2$, q2), and the triplet ($\pi 3$, $z3$, q4). When a motif indicated by a synthon completion action is added, an interface atom indicated by the synthon completion action and an attached atom for the synthon completion action may be used as a connection node. The interface atom indicated by the synthon completion action and the attached atom for the synthon completion action are a same atom in the reactant molecules.

In other words, the reactant prediction process includes an editing stage and an adding motif stage. The editing stage describes bond and atomic changes from the product molecule to synthons, that is, a synthon prediction process, while the adding motif stage completes generation of reactants by adding appropriate motifs to the synthons.

In the editing stage, an inputted molecule graph is first encoded by a GNN, to obtain an output of the GNN; then, if a $t^{th}$ action is an editing action, an RGG performs action prediction based on an output of the GNN corresponding to the $t^{th}$ action and a hidden state outputted by a previous node; and if the $t^{th}$ action is an editing complete action or a synthon completion action, the RGG performs action prediction based on the output of the GNN corresponding to the $t^{th}$ action, an attached atom corresponding to the $t^{th}$ action, and the hidden state outputted by the previous node. In other words, in the editing stage, the RNN gradually predicts the editing sequence when the editing complete action is obtained through prediction, and ends the editing stage and starts the adding motif stage. In the adding motif stage, the RNN sequentially adds motifs until all attached atoms are traversed.

In an example of FIG. 4, the first editing action is applied to a chemical bond S=O, and a new chemical bond type is none, to indicate to delete the chemical bond. In the synthon completion actions, an interface atom (q1, q2, and q3) in a motif and an attached atom (a1, a2, and a3) in a synthon/an intermediate indicate a same atom, and the interface atom and the attached atom are merged into a single atom when the motif is attached to the synthon/the intermediate. For example, when z1 is added based on the triplet ($\pi 3$, z1, q1), a1 and q1 are a same atom (namely, an atom S) in the reactant molecules; similarly, when z2 is added based on the triplet ($\pi 3$, z2, q2), a2 and q2 are a same atom (namely, an atom O) in the reactant molecules; and after z2 is added, the interface atom q3 changes to the attached atom a3, and in this case, when z3 is added based on the triplet ($\pi 3$, z3, q4), a3 and q4 are a same atom (namely, an atom C) in the reactant molecules.

It is to be understood that, FIG. 3 and FIG. 4 are examples of this application, and are not to be construed as a limitation to this application.

For example, in other alternative embodiments, the conversion path may not include the start action or the editing complete action.

FIG. 5 is a schematic flowchart of a method 300 for training a reverse reaction prediction model according to an embodiment of this application. The training method 300 may be performed by any electronic device having a data processing capability. For example, the electronic device may be implemented as a server. The server may be an independent physical server, or may be a server cluster including a plurality of physical servers or a distributed system, or may be a cloud server providing basic cloud computing services, such as a cloud service, a cloud database, cloud computing, a cloud function, cloud storage, a network service, cloud communication, a middleware service, a domain name service, a security service, big data, and an artificial intelligence platform. The server may be connected directly or indirectly through a wired or wireless communication method, which is not limited in this application. For ease of description, the prediction method provided in this application is described below by using an apparatus for training a reverse reaction prediction model as an example.

As shown in FIG. 5, the training method 300 includes the following steps.

S310: Perform feature extraction on a product molecule, to obtain a feature of the product molecule.

S320: Predict a conversion path between the product molecule and a plurality of reactant molecules by inputting the feature of the product molecule into a reverse reaction prediction model,

the conversion path including an editing sequence and a synthon completion sequence; each editing action in the editing sequence being configured for indicating an constituent and a target state, and the constituent being an atom or a chemical bond in the product molecule; and for a plurality of synthons of the product molecule obtained by using the editing sequence, the synthon completion sequence including at least one synthon completion action corresponding to each synthon in the plurality of synthons, each synthon completion action in the at least one synthon completion action being configured for indicating a motif and an interface atom, and the motif including a plurality of atoms or atomic edges for connecting the plurality of atoms; and

S330: Train the reverse reaction prediction model based on a loss between the conversion path and a training path.

Based on the foregoing technical solutions, by introducing a conversion path between a product molecule and a plurality of reactant molecules, a reverse reaction prediction model can learn a potential relationship between two sub-tasks: synthon prediction and synthon completion. In this way, generalization performance of the model is greatly improved, reactant molecule prediction complexity can be reduced, and generalization performance of reactant molecule prediction can be improved.

In addition, by introducing a motif and designing the motif as a structure including a plurality of atoms or atomic edges for connecting the plurality of atoms, a short and accurate conversion path can be reasonably constructed. In this way, an excessively long length of a synthon completion sequence is avoided, reactant molecule prediction difficulty is reduced, and reactant molecule prediction accuracy is improved, so that reactant molecule prediction performance can be improved.

In some embodiments, before S320, the method 300 may further include:
obtaining a candidate reactant molecule corresponding to the product molecule; obtaining a motif dictionary by comparing molecular structures of the product molecule and the candidate reactant molecule; and obtaining the training path based on the motif dictionary.

For example, the candidate reactant molecule may be all reactant molecules of the product molecule. To be specific, the candidate reactant molecule may be configured for generating all the reactant molecules of the product molecule.

In some embodiments, a connection tree is constructed based on the motif dictionary, where the connection tree includes a tree structure using the plurality of synthons as root nodes and using motifs in the motif dictionary as sub-nodes; and a shortest path is determined as the training path in a manner of traversing the connection tree.

A reactant molecule may be decomposed into a synthon and a motif, where the synthon is a molecular fragment obtained after a chemical bond in a product molecule graph is disconnected, and the motif is a sub-graph of a reactant. Therefore, a connection relationship between the synthon and the motif is maintained by constructing the connection tree. The connection tree indicates the synthon and the motif as a hierarchical tree structure, where the synthon is a root node, and the motif is used as a sub-node. An edge between two nodes in the connection tree indicates that two sub-graphs are directly connected in the reactant molecule graph, where a triplet of an attached atom, a motif, and an interface atom may be used to indicate each edge.

In this embodiment, a tree structure (namely, the connection tree) is constructed to indicate the connection relationship between the synthon and the motif, and the connection tree may be used to provide an effective strategy for constructing the training path, to reduce training complexity.

For example, the entire connection tree may be traversed through depth-first search, and a shortest path after traversing is determined as the training path.

The depth-first traversing manner may be traversing starting from a specified vertex v in the tree structure in the following manner:

1. Visit the vertex v.

2. Sequentially start from neighbor points of v that are not visited, and perform depth-first traversing on the tree structure, until vertices in the tree structure that are connected to v by a path have been visited.

3. If there are still unvisited vertices in the tree structure in this case, start from an unvisited vertex, and repeat the depth-first traversing, until all vertices in the tree structure have been visited.

A search strategy followed by the depth-first search is to search the tree as "deeply" as possible. A basic idea thereof is as follows: to find a solution to a problem, a possible situation is first selected to explore forward (a sub-node). In an exploration process, once it is found that an original choice does not meet a requirement, a parent node is traced back to, and another node is selected again to continue to explore forward. The process is repeated until an optimal solution is obtained. In other words, the depth-first search starts from a vertex V0 and goes to the end along a path; and returns, if it is found that a target solution cannot be reached, to a previous node and then starts from another path and goes to the end. Such a concept of trying to go as deep as possible is a concept of depth first.

In some embodiments, molecular fragments other than the plurality of synthons in the candidate reactant molecule are determined as a plurality of candidate sub-graphs; if a first candidate sub-graph in the plurality of candidate sub-graphs includes a first atom and a second atom, and the first atom and the second atom belong to different rings, a chemical bond between the first atom and the second atom is disconnected, to obtain a plurality of first sub-graphs; if a second candidate sub-graph in the plurality of candidate sub-graphs includes a third atom and a fourth atom that are connected to each other, one of the third atom and the fourth atom belongs to a ring, and a degree of an other atom in the third atom and the fourth atom is greater than or equal to a preset value, a chemical bond between the third atom and the fourth atom is disconnected, to obtain a plurality of second sub-graphs; and candidate sub-graphs other than the first candidate sub-graph and the second candidate sub-graph in the plurality of candidate sub-graphs, the plurality of first sub-graphs, and the plurality of second sub-graphs are determined as motifs in the motif dictionary.

For example, a product molecule may be decomposed into a group of incomplete sub-graphs, referred to as synthons. Therefore, after a suitable motif is bonded with each attached atom, the synthons may be reconstructed as a reactant molecule graph. In other words, a motif may be regarded as a sub-graph on the reactant molecule graph. Therefore, a motif extraction process in this embodiment of this application is divided into the following steps.

1. Edges that differ between the synthon and the reactant corresponding to the synthon are disconnected, to obtain in a series of sub-graphs. Interface atoms corresponding to attached atoms on the synthon are reserved on the sub-graphs.

It is to be understood that, the reactant corresponding to the synthon may include a molecule or a reactant including the synthon.

2. If there are two atoms that are connected to each other and that respectively belong to two rings on a sub-graph, a chemical bond connected between the two atoms is disconnected, to obtain two smaller sub-graphs.

It is to be understood that, the ring in embodiments of this application may be a single ring, in other words, a molecule has only one ring. Correspondingly, if there are two atoms that are connected to each other and that respectively belong to two single rings on a sub-graph, a chemical bond connected between the two atoms is disconnected, to obtain two smaller sub-graphs. In addition, the ring in embodiments of this application may be a cycloalkane, and the cycloalkane may be classified based on a quantity of carbon atoms on the ring. For example, the cycloalkane is referred to as a small ring when the quantity of carbon atoms on the ring is 3 to 4, the cycloalkane is referred to as an ordinary ring when the quantity of carbon atoms on the ring is 5 to 6, the cycloalkane is referred to as a middle ring when the quantity of carbon atoms on the ring is 7 to 12, and the cycloalkane is referred to as a large ring when the quantity of carbon atoms on the ring is greater than 12.

3. For two atoms connected to each other on a sub-graph, if one atom belongs to a ring and a degree of the other atom is greater than 1, a chemical bond connected between the two atoms is disconnected, to obtain two smaller sub-graphs.

Finally, a dictionary Z with a preset quantity of motifs may be extracted. For example, a dictionary Z with a preset quantity of motifs as 210 may be extracted.

In some embodiments, before S330, the method 300 may further include:

determining the loss between the conversion path and the training path based on the following information:

a difference between a label of a prediction action in the conversion path and a label of a training action in the training path, a difference between a score of an constituent corresponding to the prediction action and a score of an constituent corresponding to the training action, a difference between a target state corresponding to the prediction action and a target state corresponding to the training action, a difference between a motif indicated by the prediction action and a motif indicated by the training action, and a difference between an interface atom indicated by the prediction action and an interface atom indicated by the training action.

In embodiments of this application, a training target of the reverse reaction prediction model is to give a training path, to obtain a conversion path through prediction. Therefore, reverse reaction prediction is modeled as an autoregression-based molecule generation problem. To be specific, a product molecule $G_P$ is given, and for each step $t$, an autoregression model obtains a new graph structure $G_t$ based on a historical graph structure. When a reactant molecule $G_R$ is obtained through prediction, it indicates that a generation process is completed. Therefore, a generation process of the reactant molecule graph may be defined as the following joint probability-likelihood function:

$$P(G_R) = \prod_{t=1}^{N} P(G_t | G_0, \ldots, G_{t-1}) = \prod_{t=1}^{N} P(G_t | G_{<t}).$$

$G_R$ indicates the reactant molecule, N is a length of the conversion path, $G_t$ is an intermediate molecular fragment corresponding to a $t^{th}$ action, and $G_0 = G_P$.

The intermediate molecular fragment $G_t$ is not directly generated by the reverse reaction prediction model. However, the reverse reaction prediction model generates a new graph editing action, an constituent (namely, a chemical bond, an atom, or a motif), and a target state (namely, a new chemical bond type or an interface atom) of the constituent based on a historical action, and acts them to an intermediate molecular fragment in a previous step to obtain a new intermediate molecular fragment. Based on this, for a given historical object, target state, and intermediate molecular fragment, the likelihood function may be modified as:

$$P(G_R) = \prod_{t=1}^{N} P(\pi_t, o_t, \tau_t | o_{<t}, \tau_{<t}, G_{<t}).$$

For example, a cross-entropy loss $\mathcal{L}_c$ may be used to optimize the difference between the label of the prediction action in the conversion path and the label of the training action in the training path, the difference between the target state corresponding to the prediction action and the target state corresponding to the training action, the difference between the motif indicated by the prediction action and the motif indicated by the training action, and the difference between the interface atom indicated by the prediction action and the interface atom indicated by the training action;

and a binary cross-entropy loss $\mathcal{L}_b$ may be used to optimize the difference between the score of the constituent corresponding to the prediction action and the score of the constituent corresponding to the training action.

For example, the loss between the conversion path and the training path may be determined by using the following formula:

$$\mathcal{L} = \sum^{N_1+N_2} \mathcal{L}_c(\hat{a}, a) + \sum^{N_1}[\sum_{i=0}^{n+m-1} \mathcal{L}_b(\hat{s}_i, s_i) + \mathcal{L}_c(\hat{r}_b, r_b)] + \sum^{N_2}[\mathcal{L}_c(\hat{z}, z) + \mathcal{L}_c(\hat{q}, q)].$$

$N_1$ indicates a length of the editing sequence or a length of a sequence formed by the editing sequence and the editing complete action, and $N_2$ indicates a length of the editing sequence and the synthon completion sequence.

It is to be understood that, for an implementation of S320 in the training method 300, refer to an implementation of S220 in the prediction method 200. To avoid repetition, details are not described herein again.

It is to be understood that, the preferred implementations of this application are described in detail above with reference to the accompanying drawings. However, this application is not limited to the specific details in the foregoing implementations, a plurality of simple deformations may be made to the technical solution of this application within a range of the technical concept of this application, and these simple deformations fall within the protection scope of this application.

For example, the specific technical features described in the above specific implementations may be combined in any suitable manner without contradiction. To avoid unnecessary repetition, various possible combinations are not further described in this application. For another example, various different implementations of this application may alternatively be combined randomly. Such combinations are also to be considered as the content disclosed in this application provided that these combinations do not depart from the concept disclosed by this application.

For example, to reduce convergence difficulty, a teacher-forcing strategy may be used to train a model.

An RNN includes two training modes, namely, a free-running mode and a teacher-forcing mode. In the free-running mode, an output of a previous state is used as an input of a next state. A working principle of the teacher-forcing mode is as follows: at a moment t of a training process, an expected output or actual output y(t) of a training data set is used as an input x(t+1) of a next time step rather than an output h(t) generated by the model.

For another example, alternatively, after pre-training is performed based on a motif dictionary, fine-tuning may be performed on a benchmark data set.

For another example, a hyperparameter in the reverse reaction prediction model, such as a quantity of layers of a GNN and a quantity of layers of a GRU, may be adjusted.

It is also to be understood that, order numbers of the foregoing processes do not mean execution orders in method embodiments of this application. The execution orders of the processes are to be determined based on functions and internal logic of the processes, and are not to constitute any limitation on implementation processes of embodiments of this application.

The methods provided in the embodiments of this application is described above, and apparatuses provided in the embodiments of this application is described below.

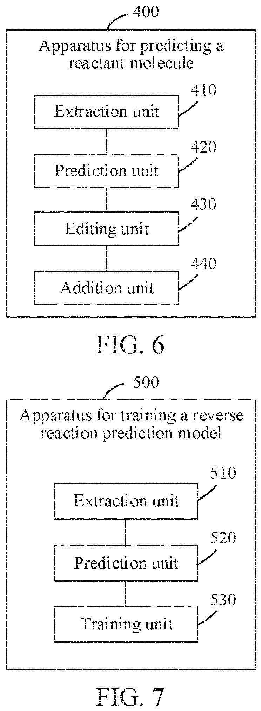

FIG. 6 is a schematic block diagram of an apparatus 400 for predicting a reactant molecule according to an embodiment of this application.

As shown in FIG. 6, the apparatus 400 for predicting a reactant molecule may include:

an extraction unit 410, configured to perform feature extraction on a product molecule, to obtain a feature of the product molecule;

a prediction unit 420, configured to predict a conversion path between the product molecule and a plurality of reactant molecules by inputting the feature of the product molecule into a reverse reaction prediction model, the conversion path including an editing sequence and a synthon completion sequence; the editing sequence being a sequence formed by editing actions, and the synthon completion sequence being a sequence formed by synthon completion actions.

an editing unit 430, configured to edit, for each editing action in the editing sequence, an constituent indicated by the respective editing action based on a target state indicated by the respective editing action, to obtain a plurality of synthons corresponding to the product molecule, the constituent being an atom or a chemical bond in the product molecule; and

an addition unit 440, configured to, for each of the plurality of synthons, determine an interface atom indicated by the at least one synthon completion action corresponding to the respective synthon and comprised in the synthon completion sequence, and add a motif indicated by the at least one synthon completion action, to obtain a reactant molecules corresponding to the respective synthon, the motif including a plurality of atoms or atomic edges for connecting the plurality of atoms.

In some embodiments, the prediction unit 420 is specifically configured to:

obtain an input feature of a $t^{th}$ action based on a $(t-1)^{th}$ action obtained through prediction of the reverse reaction prediction model, where t is an integer greater than 1; and

predict the $t^{th}$ action based on the input feature corresponding to the $t^{th}$ action and a hidden feature corresponding to the $t^{th}$ action, and obtain the conversion path when an action obtained through prediction of the reverse reaction prediction model is a synthon completion action and all attached atoms on the plurality of synthons and all attached atoms on motifs added for the plurality of synthons have been traversed, where

the hidden feature of the $t^{th}$ action is related to an action predicted by the reverse reaction prediction model before the $t^{th}$ action.

In some embodiments, the prediction unit 420 is specifically configured to:

obtain, based on a beam search manner of a hyperparameter k, k first prediction results with highest scores from prediction results of the $(t-1)^{th}$ action; and

determine k first input features corresponding to the $t^{th}$ action based on the k first prediction results; and

the obtaining the conversion path includes:

predicting the $t^{th}$ action based on each first input feature in the k first input features and the hidden feature corresponding to the $t^{th}$ action, and obtaining, based on the beam search manner of the hyperparameter k, k second prediction results with highest scores from 2k prediction results obtained through prediction;

determining k second input features corresponding to a $(t+1)^{th}$ action based on the k second prediction results; and

predicting the $(t+1)^{th}$ action based on each second input feature in the k second input features and a hidden feature corresponding to the $(t+1)^{th}$ action, and obtaining the conversion path when an action obtained through prediction of the reverse reaction prediction model is a synthon completion action and all attached atoms on the plurality of synthons and all attached atoms on motifs added for the plurality of synthons have been traversed.

In some embodiments, the prediction unit 420 is specifically configured to:

determine, if the $(t-1)^{th}$ action is the editing action, the input feature of the $t^{th}$ action based on a feature of a sub-graph obtained through editing by using the $(t-1)^{th}$ action; and predict, based on the input feature of the $t^{th}$ action and the hidden feature of the $t^{th}$ action, an constituent and a target state that are indicated by the $t^{th}$ action by using the reverse reaction prediction model, and obtain the editing sequence when an action obtained through prediction of the reverse reaction prediction model is a final editing action in the editing sequence; and

determine, if the $(t-1)^{th}$ action is a final editing action or the synthon completion action, the input feature of the $t^{th}$ action based on a feature of a sub-graph obtained through editing by using the $(t-1)^{th}$ action and a feature of an attached atom corresponding to the $(t-1)^{th}$ action; predict, based on the input feature of the $t^{th}$ action and the hidden feature of the $t^{th}$ action, a motif and an interface atom that are indicated by the $t^{th}$ action; and obtain the synthon completion sequence when an action obtained through prediction of the reverse reaction prediction model is a synthon completion action and all attached atoms on the plurality of synthons and all attached atoms on motifs added for the plurality of synthons have been traversed.

In some embodiments, the editing action is represented through the following labels: an action label configured for indicating editing, a label configured for indicating the object, and a label configured for indicating the target state; and the synthon completion action is represented through the following labels: an action label configured for indicating to perform synthon completion, a label configured for indicating the motif, and a label configured for indicating the interface atom.

In some embodiments, if the constituent indicated by the respective editing action is the atom, the target state indicated by the respective editing action is changing a quantity of charges on the atom or changing a quantity of hydrogen atoms on the atom; or if the constituent indicated by the respective editing action is the chemical bond, the target state indicated by the respective editing action is any one of the following: the chemical bond is added, the chemical bond is deleted, and the chemical bond is changed in type.

FIG. 7 is a schematic block diagram of an apparatus 500 for training a reverse reaction prediction model according to an embodiment of this application.

As shown in FIG. 7, the apparatus 500 for training a reverse reaction prediction model may include:

an extraction unit 510, configured to perform feature extraction on a product molecule, to obtain a feature of the product molecule;

a prediction unit 520, configured to predict a conversion path between the product molecule and a plurality of reactant molecules by inputting the feature of the product molecule into the reverse reaction prediction model,

the conversion path including an editing sequence and a synthon completion sequence; each editing action in the editing sequence being configured for indicating an constituent and a target state, and the constituent being an atom or a chemical bond in the product molecule; and for a plurality of synthons of the product molecule obtained by using the editing sequence, the synthon completion sequence including at least one synthon completion action corresponding to each synthon in the plurality of synthons, each synthon completion action in the at least one synthon completion action being configured for indicating a motif and an interface atom, and the motif including a plurality of atoms or atomic edges for connecting the plurality of atoms; and

a training unit 530, configured to train the reverse reaction prediction model based on a loss between the conversion path and a training path.

In some embodiments, before obtaining the conversion path, the prediction unit 520 is further configured to:

obtain a candidate reactant molecule corresponding to the product molecule;

obtain a motif dictionary by comparing molecular structures of the product molecule and the candidate reactant molecule; and

obtain the training path based on the motif dictionary.

In some embodiments, the prediction unit 520 is specifically configured to:

construct a connection tree based on the motif dictionary, where the connection tree includes a tree structure using the plurality of synthons as root nodes and using motifs in the motif dictionary as sub-nodes; and

determine a shortest path as the training path in a manner of traversing the connection tree.

In some embodiments, the prediction unit 520 is specifically configured to:

determine molecular fragments other than the plurality of synthons in the candidate reactant molecule as a plurality of candidate sub-graphs;

if a first candidate sub-graph in the plurality of candidate sub-graphs includes a first atom and a second atom, and the first atom and the second atom belong to different rings, disconnect a chemical bond between the first atom and the second atom, to obtain a plurality of first sub-graphs;

if a second candidate sub-graph in the plurality of candidate sub-graphs includes a third atom and a fourth atom that are connected to each other, one of the third atom and the fourth atom belongs to a ring, and a degree of an other atom in the third atom and the fourth atom is greater than or equal to a preset value, disconnect a chemical bond between the third atom and the fourth atom, to obtain a plurality of second sub-graphs; and

determine candidate sub-graphs other than the first candidate sub-graph and the second candidate sub-graph in the plurality of candidate sub-graphs, the plurality of first sub-graphs, and the plurality of second sub-graphs as motifs in the motif dictionary.

In some embodiments, before training the reverse reaction prediction model based on the loss between the conversion path and the training path, the training unit 530 is further configured to:

determine the loss between the conversion path and the training path based on the following information:

a difference between a label of a prediction action in the conversion path and a label of a training action in the training path, a difference between a score of an constituent corresponding to the prediction action and a score of an constituent corresponding to the training action, a difference between a target state corresponding to the prediction action and a target state corresponding to the training action, a difference between a motif indicated by the prediction action and a motif indicated by the training action, and a difference between an interface atom indicated by the prediction action and an interface atom indicated by the training action.

It is to be understood that, the apparatus device embodiment and the method embodiment may correspond to each other. For a similar description, refer to the method embodiment. To avoid repetition, details are not described herein again. Specifically, the apparatus 400 for predicting a reactant molecule may correspond to a corresponding entity in the method 200 in the embodiments of this application, and the units in the prediction apparatus 400 are respectively configured to implement corresponding procedures in the method 200. Similarly, the apparatus 500 for training a reverse reaction prediction model may correspond to a corresponding entity in the method 300 in the embodiments of this application, and the units in the training apparatus 500 are respectively configured to implement corresponding procedures in the method 300. For brevity, details are not described herein again.

It is also to be understood that, the units of the prediction apparatus 400 or the training apparatus 500 in the embodiments of this application may be separately or wholly combined into one or several other units, or one (or more) of the units herein may further be divided into multiple units of smaller functions. In this way, same operations can be implemented, and implementation of the technical effects of the embodiments of this application is not affected. The foregoing units are divided based on logical functions. In an actual application, a function of one unit may also be implemented by a plurality of units, or functions of a plurality of units are implemented by one unit. In other embodiments of this application, the prediction apparatus 400 or the training apparatus 500 may also include another unit. During practical application, these functions may also be cooperatively implemented by another unit and may be cooperatively implemented by a plurality of units. According to another embodiment of this application, a computer program (including program code) that can perform the steps in the corresponding method may be run on a general computing device, such as a general computer, which includes processing elements and storage elements such as a central processing unit (CPU), a random access memory (RAM), and a read-only memory (ROM), to construct the prediction apparatus 400 or the training apparatus 500 in the embodiments of this application and implement the methods in the embodiments of this application. The computer program may be recorded in, for example, a computer-readable storage medium, and may be loaded into the foregoing computing device by using the computer-readable storage medium, and run in an electronic device, to implement the corresponding method in embodiments of this application.

In other words, the foregoing units may be implemented in a form of hardware, or may be implemented in a form of instructions in a form of software, or may be implemented in a form of a combination of software and hardware. Specifically, steps in the foregoing method embodiments in the embodiments of this application may be completed by using a hardware integrated logical circuit in the processor, or by using instructions in a form of software. The steps of the methods disclosed with reference to the embodiments of this application may be directly performed and completed by using a hardware decoding processor, or may be performed and completed by using a combination of hardware and software in the decoding processor. In one embodiment, the software may be located in a mature storage medium in the art, such as a random access memory, a flash memory, a read-only memory, a programmable read-only memory, an electrically erasable programmable memory, a register, or the like. The storage medium is located in a memory, and the processor reads information in the memory and completes the steps in the foregoing method embodiments in combination with the hardware in the processor.

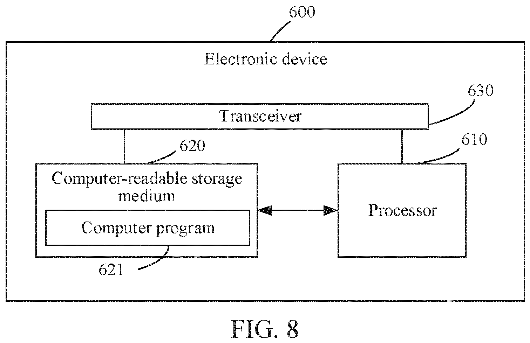

FIG. 8 is a schematic structural diagram of an electronic device 600 according to an embodiment of this application.

As shown in FIG. 8, the electronic device 600 includes at least a processor 610 and a computer-readable storage medium 620. The processor 610 may be connected with the computer-readable storage medium 620 through a bus or other manners. The computer-readable storage medium 620 is configured to store a computer program 621. The computer program 621 includes computer instructions. The processor 610 is configured to execute the computer instructions stored in the computer-readable storage medium 620. The processor 610 is a computing core and a control core of the electronic device 600, is adapted to implement one or more computer instructions, and is specifically adapted to load and execute the one or more computer instructions to implement a corresponding method procedure or a corresponding function.

In an example, the processor 610 may also be referred to as a central processing unit (CPU). The processor 610 may include, but not limited to: a general purpose processor, a digital signal processor (DSP), an application specific integrated circuit (ASIC), a field programmable gate array (FPGA), another programmable logical device, discrete gate or transistor logical device, or discrete hardware component, or the like.

In an example, the computer-readable storage medium 620 may be a high-speed RAM, or a non-volatile memory, such as at least one disk memory. In one embodiment, the computer-readable storage medium may be at least one computer-readable storage medium far away from the processor 610. Specifically, the computer-readable storage medium 620 includes, but not limited to: a volatile memory and/or a non-volatile memory. The non-volatile memory may be a read-only memory (ROM), a programmable ROM (PROM), an erasable programmable read-only memory (EPROM), an electrically EPROM (EEPROM), or a flash memory. The volatile memory may be a random access memory (RAM), and is used as an external cache. Through exemplary but not limitative description, many forms of RAMs may be used, for example, a static random access memory (SRAM), a dynamic random access memory (DRAM), a synchronous dynamic random access memory (SDRAM), a double data rate synchronous dynamic random access memory (DDR SDRAM), an enhanced synchronous dynamic random access memory (ESDRAM), a synch link dynamic random access memory (SLDRAM) and a direct rambus random access memory (DR RAM).

As shown in FIG. 8, the electronic device 600 may further include a transceiver 630.

The processor 610 may control the transceiver 630 to communicate with another device, and specifically, may send information or data to the another device, or receive information or data sent by the another device. The transceiver 630 may include a transmitter and a receiver. The transceiver 630 may further include an antenna, and a quantity of the antenna can be one or more.

It is to be understood that, various components of the electronic device 600 are connected to each other by using a bus system. In addition to including a data bus, the bus system further includes a power bus, a control bus, and a status signal bus.

In an implementation, the electronic device 600 may be any electronic device having a data processing function. The computer-readable storage medium 620 stores a first computer instruction. The processor 610 loads and executes the first computer instruction stored in the computer-readable storage medium 620, to implement corresponding steps of the method embodiment in FIG. 1. During specific implementation, the first computer instruction in the computer-readable storage medium 620 is loaded by the processor 610 to perform the corresponding steps. To avoid repetition, details are not described herein again.

According to another aspect of this application, an embodiment of this application further provides a computer-readable storage medium (memory), and the computer-readable storage medium is a memory device in the electronic device 600 and is configured to store programs and data, for example, the computer-readable storage medium 620. It may be understood that, the computer-readable storage medium 620 herein may include an internal storage medium of the electronic device 600 and certainly may also include an extended storage medium supported by the electronic device 600. The computer-readable storage medium provides storage space, and the storage space stores an operating system of the electronic device 600. Moreover, computer instructions suitable for the processor 610 to load and execute is further stored in the memory space. The computer instructions may be one or more computer programs 621 (including program code).

According to another aspect of this application, an embodiment of this application further provides a computer program product or a computer program, including computer instructions, the computer instructions being stored in a computer-readable storage medium, for example, the computer programs 621. In this case, the electronic device 600 may be a computer. The processor 610 reads the computer instructions from the computer-readable storage medium 620, and the processor 610 executes the computer instructions, to enable the computer to perform the method provided in the various optional manners.

In other words, when software is used for implementation, all or some of the embodiments may be implemented in a form of a computer program product. The computer program product includes one or more computer instructions. When the computer program instructions are loaded and executed on a computer, procedures in the embodiments of this application or functions in the embodiments of this application are all or partially implemented. The computer may be a general-purpose computer, a special-purpose computer, a computer network, or another programmable apparatus. The computer instructions may be stored in a computer-readable storage medium, or transmitted from one computer-readable storage medium to another computer-readable storage medium. For example, the computer-executable instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner.

A person of ordinary skill in the art may notice that the exemplary units and procedure steps described with reference to the embodiments disclosed in this specification can be implemented in electronic hardware, or a combination of computer software and electronic hardware. Whether the functions are performed by hardware or software depends on particular applications and design constraints of the technical solutions. A person skilled in the art may use different methods to implement the described functions for each particular application, but it is not to be considered that the implementation goes beyond the scope of this application.

The foregoing content is merely specific implementations of this application, but is not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1.   A method for predicting a reactant molecule, comprising:

performing feature extraction on a product molecule, to obtain a feature of the product molecule;
predicting a conversion path between the product molecule and a set of to-be-predicted reactant molecules by inputting the feature of the product molecule into a reverse reaction prediction model, the conversion path comprising an editing sequence and a synthon completion sequence, the editing sequence comprising a plurality of editing actions each indicating both a constituent of the product molecule and a target state of the constituent, the constituent being an atom or a chemical bond of the product molecule, the synthon completion sequence comprising a plurality of synthon complete actions each indicating both an interface atom and a corresponding motif, the motif comprising a plurality of atoms or atomic edges for connecting the plurality of atoms;
performing the plurality of editing actions to edit the respective constituent into the corresponding target state indicated by the respective editing action, in order to obtain a plurality of synthons corresponding to the product molecule; and
performing, for each of the plurality of synthons, at least one synthon completion action corresponding to the respective synthon, to add the motif indicated by the at least one corresponding synthon completion action according to the corresponding interface atom, to obtain a plurality of predicted reactant molecules each corresponding to a respective one of the plurality of synthons.

2.   The method according to claim 1, wherein the predicting a conversion path between the product molecule and a set of to-be-predicted reactant molecules by inputting the feature of the product molecule into a reverse reaction prediction model comprises:

obtaining an input feature of a $t^{th}$ action based on a $(t-1)^{th}$ action obtained through prediction of the reverse reaction prediction model, wherein t is an integer greater than 1; and
predicting the $t^{th}$ action based on an input feature corresponding to the $t^{th}$ action and a hidden feature corresponding to the $t^{th}$ action, and obtaining the conversion path when an action obtained through prediction of the reverse reaction prediction model is a synthon completion action and all attached atoms on the plurality of synthons and all attached atoms on motifs added for the plurality of synthons have been traversed, wherein the hidden feature of the $t^{th}$ action is related to an action predicted by the reverse reaction prediction model before the $t^{th}$ action.

3.   The method according to claim 2, wherein the obtaining an input feature of a $t^{th}$ action based on a $(t-1)^{th}$ action obtained through prediction of the reverse reaction prediction model comprises:

obtaining, based on a beam search manner of a hyperparameter k, k first prediction results with highest scores from prediction results of the $(t-1)^{th}$ action; and
determining k first input features corresponding to the $t^{th}$ action based on the k first prediction results; and
the obtaining the conversion path comprises:

predicting the $t^{th}$ action based on each first input feature in the k first input features and the hidden feature corresponding to the $t^{th}$ action, and obtaining, based on the beam search manner of the hyperparameter k, k second prediction results with highest scores from prediction results obtained through prediction;
determining k second input features corresponding to a $(t+1)^{th}$ action based on the k second prediction results; and
predicting the $(t+1)^{th}$ action based on each second input feature in the k second input features and a hidden feature corresponding to the (t+1)$^{th}$ action, and obtaining the conversion path when an action obtained through prediction of the reverse reaction prediction model is a synthon completion action and all attached atoms on the plurality of synthons and all attached atoms on motifs added for the plurality of synthons have been traversed.

4.   The method according to claim 2, wherein the obtaining the conversion path comprises:

determining, in a case that the (t-1)$^{th}$ action is the editing action, the input feature of the t$^{th}$ action based on a feature of a sub-graph obtained through editing by using the (t-1)$^{th}$ action; and
predicting, based on the input feature of the t$^{th}$ action and the hidden feature of the t$^{th}$ action, an constituent and a target state that are indicated by the t$^{th}$ action by using the reverse reaction prediction model, and obtaining the editing sequence when an action obtained through prediction of the reverse reaction prediction model is a final editing action in the editing sequence.

5.   The method according to claim 2, wherein the obtaining the conversion path comprises:

determining, in a case that the (t-1)$^{th}$ action is a final editing action or the synthon completion action, the input feature of the t$^{th}$ action based on a feature of a sub-graph obtained through editing by using the (t-1)$^{th}$ action and a feature of an attached atom corresponding to the (t-1)$^{th}$ action;
predicting, based on the input feature of the t$^{th}$ action and the hidden feature of the t$^{th}$ action, a motif and an interface atom that are indicated by the t$^{th}$ action; and obtaining the synthon completion sequence when an action obtained through prediction of the reverse reaction prediction model is a synthon completion action and all attached atoms on the plurality of synthons and all attached atoms on motifs added for the plurality of synthons have been traversed.

6.   The method according to any one of claims 1 to 5, wherein the editing action is represented through the following labels: an action label configured for indicating editing, a label configured for indicating the object, and a label configured for indicating the target state; and the synthon completion action is represented through the following labels: an action label configured for indicating to perform synthon completion, a label configured for indicating the motif, and a label configured for indicating the interface atom.

7.   The method according to any one of claims 1 to 6, wherein in a case that the constituent indicated by the respective editing action is the atom, the target state indicated by the respective editing action is changing a quantity of charges on the atom or changing a quantity of hydrogen atoms on the atom; or in a case that the constituent indicated by the respective editing action is the chemical bond, the target state indicated by the respective editing action is any one of the following: the chemical bond is added, the chemical bond is deleted, and the chemical bond is changed in type.

8.   A method for training a reverse reaction prediction model, comprising:

performing feature extraction on a product molecule, to obtain a feature of the product molecule;
predicting a conversion path between the product molecule and a plurality of reactant molecules by inputting the feature of the product molecule into the reverse reaction prediction model,
the conversion path comprising an editing sequence and a synthon completion sequence; each editing action in the editing sequence being configured for indicating an constituent and a target state, and the constituent being an atom or a chemical bond in the product molecule; and for a plurality of synthons of the product molecule obtained by using the editing sequence, the synthon completion sequence comprising at least one synthon completion action corresponding to each synthon in the plurality of synthons, each synthon completion action in the at least one synthon completion action being configured for indicating a motif and an interface atom, and the motif comprising a plurality of atoms or atomic edges for connecting the plurality of atoms; and
training the reverse reaction prediction model based on a loss between the conversion path and a training path.

9.   The method according to claim 8, wherein before the obtaining the conversion path, the method further comprises:

obtaining a candidate reactant molecule corresponding to the product molecule;
obtaining a motif dictionary by comparing molecular structures of the product molecule and the candidate reactant molecule; and
obtaining the training path based on the motif dictionary.

10. The method according to claim 9, wherein the obtaining the training path based on the motif dictionary comprises:

constructing a connection tree based on the motif dictionary, wherein the connection tree comprises a tree structure using the plurality of synthons as root nodes and using motifs in the motif dictionary as sub-nodes; and determining a shortest path as the training path in a manner of traversing the connection tree.

11. The method according to claim 9 or 10, wherein the obtaining a motif dictionary by comparing molecular structures of the product molecule and the candidate reactant molecule comprises:

determining molecular fragments other than the plurality of synthons in the candidate reactant molecule as a plurality of candidate sub-graphs;
in a case that a first candidate sub-graph in the plurality of candidate sub-graphs comprises a first atom and a second atom, and the first atom and the second atom belong to different rings, disconnecting a chemical bond between the first atom and the second atom, to obtain a plurality of first sub-graphs;
in a case that a second candidate sub-graph in the plurality of candidate sub-graphs comprises a third atom and a fourth atom that are connected to each other, one of the third atom and the fourth atom belongs to a ring, and a degree of an other atom in the third atom and the fourth atom is greater than or equal to a preset value, disconnecting a chemical bond between the third atom and the fourth atom, to obtain a plurality of second sub-graphs; and
determining candidate sub-graphs other than the first candidate sub-graph and the second candidate sub-graph in the plurality of candidate sub-graphs, the plurality of first sub-graphs, and the plurality of second sub-graphs as motifs in the motif dictionary.

12. The method according to any one of claims 8 to 11, wherein before the training the reverse reaction prediction model based on a loss between the conversion path and a training path, the method further comprises:
determining the loss between the conversion path and the training path based on the following information:
a difference between a label of a prediction action in the conversion path and a label of a training action in the training path, a difference between a score of an constituent corresponding to the prediction action and a score of an constituent corresponding to the training action, a difference between a target state corresponding to the prediction action and a target state corresponding to the training action, a difference between a motif indicated by the prediction action and a motif indicated by the training action, and a difference between an interface atom indicated by the prediction action and an interface atom indicated by the training action.

13. An apparatus for predicting a reactant molecule, comprising:

an extraction unit, configured to perform feature extraction on a product molecule, to obtain a feature of the product molecule;
a prediction unit, configured to predict a conversion path between the product molecule and a set of to-be-predicted reactant molecules by inputting the feature of the product molecule into a reverse reaction prediction model, the conversion path comprising an editing sequence and a synthon completion sequence, the editing sequence comprising a plurality of editing actions each indicating both a constituent of the product molecule and a target state of the constituent, the constituent being an atom or a chemical bond of the product molecule, the synthon completion sequence comprising a plurality of synthon complete actions each indicating both an interface atom and a corresponding motif, the motif comprising a plurality of atoms or atomic edges for connecting the plurality of atoms;
an editing unit, configured to perform the plurality of editing actions to edit the respective constituent into the corresponding target state indicated by the respective editing action, in order to obtain a plurality of synthons corresponding to the product molecule; and
an addition unit, configured to perform, for each of the plurality of synthons, at least one synthon completion action corresponding to the respective synthon, to add the motif indicated by the at least one corresponding synthon completion action according to the corresponding interface atom, to obtain a plurality of predicted reactant molecules each corresponding to a respective one of the plurality of synthons.

14. An apparatus for training a reverse reaction prediction model, comprising:

an extraction unit, configured to perform feature extraction on a product molecule, to obtain a feature of the product molecule;
a prediction unit, configured to predict a conversion path between the product molecule and a plurality of reactant molecules by inputting the feature of the product molecule into the reverse reaction prediction model, the conversion path comprising an editing sequence and a synthon completion sequence; each editing action in the editing sequence being configured for indicating an constituent and a target state, and the constituent being an atom or a chemical bond in the product molecule; and for a plurality of synthons of the product molecule obtained by using the editing sequence, the synthon completion sequence comprising at least one synthon completion action corresponding to each synthon in the plurality of synthons, each synthon completion action in the at least one synthon completion action being configured for indicating a motif and an interface atom, and the motif comprising a plurality of atoms or atomic edges for connecting the plurality of atoms; and
a training unit, configured to train the reverse reaction prediction model based on a loss between the conversion path and a training path.

15. An electronic device, comprising:

    a processor, suitable for executing a computer program; and
    a computer-readable storage medium, storing a computer program, the computer program, when executed by the processor, implementing the method for predicting a reactant molecule according to any one of claims 1 to 7, or the method for training a reverse reaction prediction model according to any one of claims 8 to 12.

16. A computer-readable storage medium, configured to store a computer program, the computer program enabling a computer to perform the method for predicting a reactant molecule according to any one of claims 1 to 7, or the method for training a reverse reaction prediction model according to any one of claims 8 to 12.

17. A computer program product, comprising a computer program/instructions, the computer program/instructions, when executed by a processor, implementing the method for predicting a reactant molecule according to any one of claims 1 to 7, or the method for training a reverse reaction prediction model according to any one of claims 8 to 12.

<u>100</u>

FIG. 1

<u>200</u>

Perform feature extraction on a product molecule, to obtain a feature of the product molecule ⟋ S210

Predict, based on the feature of the product molecule, a conversion path between the product molecule and a plurality of reactant molecules by using a reverse reaction prediction model, the conversion path including an editing sequence and a synthon completion sequence ⟋ S220

Edit an edited object indicated by each editing action based on an edited state indicated by each editing action in the editing sequence, to obtain a plurality of synthons corresponding to the product molecule ⟋ S230

S240: For each synthon in the plurality of synthons, add a motif indicated by each synthon completion action based on at least one synthon completion action corresponding to each synthon in the synthon completion sequence and an interface atom indicated by each synthon completion action in the at least one synthon completion action, to obtain a plurality of reactant molecules corresponding to the plurality of synthons, the motif including a plurality of atoms or atomic edges for connecting the plurality of atoms ⟋ S240

## FIG. 2

FIG. 3

FIG. 4

300

| Perform feature extraction on a product molecule, to obtain a feature of the product molecule | S310 |

| Predict, based on the feature of the product molecule, a conversion path between the product molecule and a plurality of reactant molecules by using a reverse reaction prediction model | S320 |

| Train the reverse reaction prediction model based on a loss between the conversion path and a training path | S330 |

FIG. 5

400

Apparatus for predicting a
reactant molecule

Extraction unit — 410

Prediction unit — 420

Editing unit — 430

Addition unit — 440

# FIG. 6

500

Apparatus for training a reverse
reaction prediction model

Extraction unit — 510

Prediction unit — 520

Training unit — 530

# FIG. 7

600

**Electronic device**

630

Transceiver

620

610

Computer-readable storage medium

Processor

Computer program

621

FIG. 8

## INTERNATIONAL SEARCH REPORT

| International application No. |
|---|
| **PCT/CN2023/096605** |

**A. CLASSIFICATION OF SUBJECT MATTER**

G16C20/30(2019.01)i

According to International Patent Classification (IPC) or to both national classification and IPC

**B. FIELDS SEARCHED**

Minimum documentation searched (classification system followed by classification symbols)

IPC:G16C、G16B、G06N

Documentation searched other than minimum documentation to the extent that such documents are included in the fields searched

Electronic data base consulted during the international search (name of data base and, where practicable, search terms used)

CNTXT, CNKI, ENTXT, DWPI: 腾讯, 反应物, 分子, 逆向, 预测, 推理, 评估, 合成子, 原子, 补全, 边, reactant, molecular, inverse, prediction, reasoning, evaluation, synthon, atom, completion, edge

**C. DOCUMENTS CONSIDERED TO BE RELEVANT**

| Category* | Citation of document, with indication, where appropriate, of the relevant passages | Relevant to claim No. |
|---|---|---|
| PX | CN 115240786 A (TENCENT TECHNOLOGY (SHENZHEN) CO., LTD.) 25 October 2022 (2022-10-25) <br> claims 1-16 | 1-17 |
| A | CN 111524557 A (TENCENT TECHNOLOGY (SHENZHEN) CO., LTD.) 11 August 2020 (2020-08-11) <br> entire document | 1-17 |
| A | CN 113782109 A (YANTAI GUOGONG INTELLIGENT TECHNOLOGY CO., LTD.) 10 December 2021 (2021-12-10) <br> entire document | 1-17 |
| A | CN 113990405 A (SHANGHAI WUXI APPTEC NEW DRUG DEVELOPMENT CO., LTD.) 28 January 2022 (2022-01-28) <br> entire document | 1-17 |
| A | KR 20210147862 A (SAMSUNG ELECTRONICS CO., LTD.; KOREA ADVANCED INSTITUTE OF SCIENCE AND TECHNOLOGY) 07 December 2021 (2021-12-07) <br> entire document | 1-17 |

☑ Further documents are listed in the continuation of Box C.  ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **15 August 2023** | **30 August 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/096605**

**C.    DOCUMENTS CONSIDERED TO BE RELEVANT**

| Category* | Citation of document, with indication, where appropriate, of the relevant passages | Relevant to claim No. |
|---|---|---|
| A | KR 20220022059 A (ARONTIER CO., LTD.) 24 February 2022 (2022-02-24)<br>entire document | 1-17 |
| A | US 2021225462 A1 (EMD MILLIPORE CORP.) 22 July 2021 (2021-07-22)<br>entire document | 1-17 |

Form PCT/ISA/210 (second sheet) (July 2022)

**INTERNATIONAL SEARCH REPORT**
Information on patent family members

International application No.

**PCT/CN2023/096605**

| Patent document cited in search report | | | Publication date (day/month/year) | Patent family member(s) | | | Publication date (day/month/year) |
|---|---|---|---|---|---|---|---|
| CN | 115240786 | A | 25 October 2022 | None | | | |
| CN | 111524557 | A | 11 August 2020 | HK | 40028372 | A0 | 05 February 2021 |
| CN | 113782109 | A | 10 December 2021 | None | | | |
| CN | 113990405 | A | 28 January 2022 | None | | | |
| KR | 20210147862 | A | 07 December 2021 | US | 2021374536 | A1 | 02 December 2021 |
| KR | 20220022059 | A | 24 February 2022 | KR | 20220062485 | A | 17 May 2022 |
| US | 2021225462 | A1 | 22 July 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210952642